# EUROPEAN PATENT APPLICATION

(11) **EP 2 100 890 A1**
(43) Date of publication of application: **16.09.2009**
(21) Application number: 07823019.0
(22) Date of filing: 26.10.2007
(51) Int. Cl.: C07D 401/12, C07D 403/12, C07D 413/12, A61P 31/18

(54) **NOVEL POLYNITROGENATED SYSTEMS AS ANTI-HIV AGENTS**

(30) Priority: 26.10.2006 ES 200602764
(71) Applicant: Institut Quimic De Sarria Cets, 08017 Barcelona (ES)
(72) Inventor: PETTERSSON SALOM, Sofia, 08870 Sitges (Barcelona) (ES); ROS BLANCO, Laia, 08037 Barcelona (ES); TEIXIDO CLOSA, Jordi, 08302 Mataro (Barcelona) (ES); BATLLORI AGUILA, Xavier, 08016 Barcelona (ES); PUIG DE LA BELLACASA CAZORLA, Raimon, 08008 Barcelona (ES); BORRELL BILBAO, Jose Ignacio, 08031 Barcelona (ES); NONELL MARRUGAT, Santiago, 08190 Sant Cugat del Valles (Barcelona) (ES); RABAL GRACIA, María Obdulia, 22130 Peralta de Alcofea (Huesca) (ES); PÉREZ NUENO, Violeta, 08013 Barcelona (ES); ESTÉ ARAQUE, José, 08391 Tiana (Barcelona) (ES); CLOTET CODINA, Imma, 08140 Caldes de Montbui (Barcelona) (ES); ARMAND UGÓN, Mercedes, 07703 Mao (Menorca) (ES)
(74) Representative: Carpintero Lopez, Francisco
(86) International application number: PCT/ES2007/000613
(87) International publication number: WO 2008/049950

(57) **Abstract**

The present invention describes the synthesis of the compounds of formulae (1), (2) and (3) in which m and n, which may be identical or different, may have the values 0, 2, 3, 4, 5 and 6; Z and Y, which may be identical or different, represent a nitrogenated cyclic system bonded by nitrogen or by one of the ring carbons or an NR¹R² system; and X is selected from the group consisting of hydrogen, C1-C12 alkyl, substituted alkyl, C3-C12 aryl, amino, alkylamino, nitro, hydroxy, alkoxy, halogen, carboxy or carboxamido. The use of the compounds of formulae (1), (2) and (3) as anti-HIV agents in the treatment of Acquired Immune Deficiency Syndrome (AIDS) is described.

## Description

### Field of the Art

The present invention relates to compounds of formulae (1), (2) and (3) and to their pharmaceutically acceptable salts, hydrates, solvates, esters and metal complexes of said structures, to their use as anti-HIV agents in the treatment of Acquired Immune Deficiency Syndrome (AIDS), to the process for obtaining them and to the synthesis intermediates used therein.

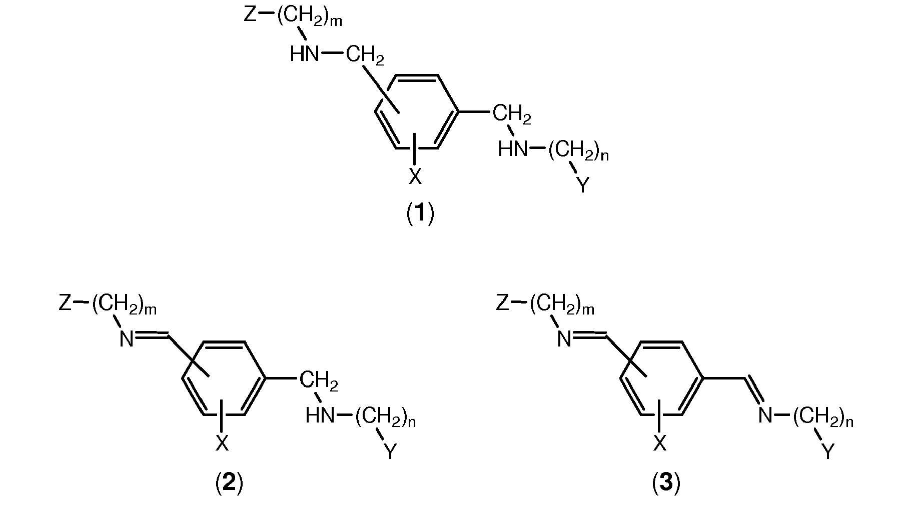

### State of the Art

In the fight against Acquired Immune Deficiency Syndrome (AIDS), the main therapeutic targets include all the phases of the biological cycle of the Human Immunodeficiency Virus (HIV) for the purpose of blocking, reducing or cancelling each of the key steps of the HIV reproductive cycle. One of the main difficulties in antiretroviral treatment is the fast evolution of the virus, which allows it to become resistant to the drugs which are used to treat it. Another difficulty lies in the side effects on the host, such that, to prevent them, the drugs must be specific against the viral proteins or enzymes and not against the reproduction mechanism.

Current antiretroviral therapy consists of combinations of two families of compounds: reverse transcriptase inhibitors (RTI) and protease inhibitors (PI), both aimed at specific enzymes produced by HIV.

The step of binding and fusing the virus to the host cell is an interesting target in chemotherapy against HIV. HIV needs a primary receptor (CD4) and chemokine receptors (CXCR4 or CCR5) as co-receptors to be fused to the cell. Chemokine receptor CXCR4 is a co-receptor for the entry of T-tropic HIV strains whereas CCR5 is a co-receptor for M-tropic strains. Therefore, the compounds which interact with the entry co-receptors could be good possible drugs against the entry of HIV.

Small chemokine receptor inhibitor molecules have been identified (Moore et al., Nat Rev Mol Cell Biol, 2000, 1, 40). The agents which block CXCR4 include small peptides such as Allelix-40-4C, T22 and analogs thereof (Doranz et al., J Exp Med, 1997, 186, 1395; Murakami et al., J Exp Med, 1997, 186, 1389); peptoids such as CGP64222 and arginine conjugates (Cabrera et al., Antiviral Research, 2002, 53, 1; Cabrera et al., AIDS Res Hum Retroviruses, 2000, 16, 627; Daelemans et al., Mol Pharmacol, 2000, 57, 116); and bicyclams (Bridger et al., J Med Chem, 1995, 38, 366; Este et al., Mol Pharmacol, 1999, 55, 67; Donzella et al., Nat Med, 1998, 4, 72; Schols et al., J Exp Med, 1997, 186, 1383). Several compounds such as Tak 779, the new derivative of spiro diketopiperazine E913 (Maeda et al., J Biol Chem, 2001, 13, 13), monoclonal antibodies such as 2D7 or PRO140 (Trkola et al., J Virol, 2001, 75, 579) and low-molecular weight compounds such as SCH-D have proven to be effective in blocking the function of CCR5 and the replication of HIV (Strizki et al., Proc Natl Acad Sci USA, 2001, 98, 12718). It has also been described that gp41 peptides, such as T-20, inhibit the replication of HIV.

HIV fusion inhibitors are becoming the next generation of anti-HIV agents. The compounds under study include BMS-488043 which binds to the gp120 of HIV-1 preventing it from recognizing the receptor CD4, AMD070 which acts as a specific inhibitor of the co-receptor CXCR4, GW-873140, UK-427857 and SCH-D which are antagonists of the co-receptor CCR5.

An antagonist of CXCR4, tAMD3100 reduces by 0.8-0.9 log10 the viral load in a subject infected with a X4 strain of HIV. The virus recovered from patients who received AMD3100 showed a change in phenotype from X4 virus to R5 virus, suggesting that AMD3100 selectively blocked those viruses using CXCR4 but that it was not effective in inhibiting the *in vivo* CCR5-dependent replication of HIV (Schols et al., 9th CROI, Seattle 2002). The development of AMD3100 was abandoned in 2001 due to a possible cardiac toxicity (Hendrix et al., Antimicrob Agents Chemother, 2000, 44, 1667; Hendrix et al., J Acquir Immune Defic Syndr, 2004, 37, 1253), furthermore its lack of oral bioavailability related to its high positive charge in physiological medium could have long-term limitations for its application in anti-HIV therapy (Hatse et al., Biochem Pharmacol, 2005, 70, 752).

The current leads AMD3100, SCH-D and TAK-779, have aromatic or aliphatic spacers in polynitrogenated systems. Of all the compounds under study, bicyclams in general and AMD3100 in particular seem to be the most active. Even so, compounds with a single cyclam unit and with the spacer 1,4-phenylenebismethylene such as AMD3465 have proven to be up to 10 times more active than AMD3100 (Hatse et al., Biochem Pharmacol, 2005, 70, 752; Princen et al., J Virol, 2004, 78,12996).

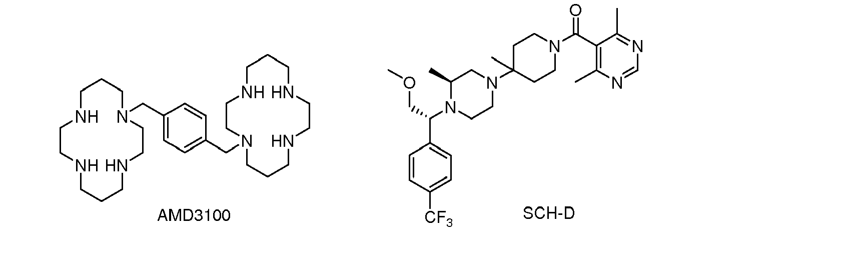

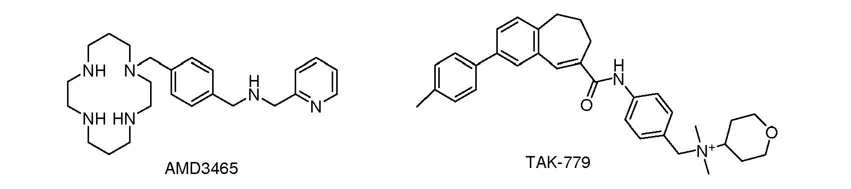

With this background, the authors of the present invention set out to obtain compounds in which both cyclam rings, too basic and possibly responsible for the toxicity observed in AMD3100, are substituted by other nitrogenated cyclic or heterocyclic systems with lower basicity and, therefore, with lower toxicity but maintaining their activity as inhibitors of the entry co-receptors CXCR4 and CCR5.

For the purpose of maintaining the benzyl nitrogen of both sides of the p-phenylene unit and the nitrogen of the selected nitrogenated heterocyclic systems at a distance similar to that existing in AMD31 00, compounds containing a spacer - (CH₂)ₙ- (n = 0, 2, 3, 4, 5, 6) between said benzyl nitrogens and the nitrogenated heterocyclic systems were designed. The latter could be bound to said spacer chain both by the nitrogen and by a ring carbon.

A literature search revealed that any system with these characteristics had been previously synthetized in a polymer chemistry environment as catalysts (Habaue et al. J. Polym. Sci., Part A: Polym. Chem. 2005, 43(8), 1635-1640) or as ligands for metal complexes (Habagami *et al.,* Jpn. Kokai Tokkyo Koho JP 2005314274 (2005)).

Consequently, virtual chemical libraries of structures of formula (1), (2) and (3) were designed and constructed, and selected by means of an "in silico" test on suitably modeled receptors CXCR4 and CCR5.

Surprisingly, the tested and synthesized compounds generally have an EC₅₀ < 10 µg/mL in an anti-HIV test in MT-4 cells, the CC₅₀ values generally being greater than 25 µg/mL, proving the validity of the hypothesis.

### Object of the Invention

The present invention relates to compounds of formulae (1), (2) and (3) and to the use thereof as anti-HIV agents in the treatment of Acquired Immune Deficiency Syndrome (AIDS), to the process for obtaining them and to the synthesis intermediates used therein.

### Description of the Invention

The compounds object of the present invention are selected from the formulae (1), (2) and (3) described below.

The compounds of formula (1) have the following structure: 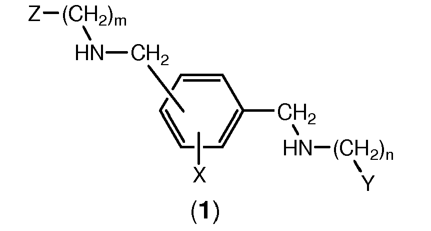 wherein:
the substituent -CH₂-NH-(CH₂)ₘ-Z is *meta* or *para* to the substituent -CH₂-NH-(CH₂)ₙ-Y wherein m and n, which may be identical or different, may have the values 0, 2, 3, 4, 5 and 6;
   Z and Y, which may be identical or different, represent a nitrogenated heterocyclic system bonded by nitrogen (the corresponding m or n being greater than or equal to 2) or by one of the ring carbons; a substituted nitrogenated heterocyclic system bonded by nitrogen (the corresponding m or n being greater than or equal to 2) or by one of the ring carbons; an NR¹R² group (the corresponding m or n being greater than or equal to 2) wherein R¹ and R² are independently selected from among hydrogen, C₁-C₁₂ alkyl, substituted alkyl, C₃-C₁₂ aryl, substituted C₃-C₁₂ aryl, cycloalkyl and substituted cycloalkyl
   X is selected from the group consisting of hydrogen, C₁-C₁₂ alkyl, substituted alkyl, C₃-C₁₂ aryl, amino, alkylamino, nitro, hydroxy, alkoxy, halogen, carboxy or carboxamido;
   and pharmaceutically acceptable salts, hydrates, solvates, esters and metal complexes of said structure.

The term "nitrogenated heterocyclic system" means an aromatic or nonaromatic monocyclic or polycyclic system containing between 5 and 24 atoms of which between 1 and 4 are nitrogen atoms and between 0 and 4 are oxygen atoms. Preferred compounds among the compounds of formula (1) of the present invention are those in which the nitrogenated heterocyclic system bonded by nitrogen or by one of the ring carbons is selected from among: pyrrolidine, piperazine, piperidine, morpholine, azacycloheptane, diazacycloheptane, azacyclotridecane, diazacyclooctane, pyrrole, imidazole, thiazole, prazole, pyridine, pyrimidine.

The term "substituted nitrogenated heterocyclic system" means any of the previous nitrogenated heterocyclic systems substituted by one or several C₁-C₁₂ alkyl chains in the ring carbons or in the ring nitrogens if these are not bound to the - (CH₂)ₙ- chain or to the -(CH₂)ₘ- chain. Preferred compounds among the compounds of formula (1) of the present invention are those in which the substituted nitrogenated heterocyclic system bonded by nitrogen or by one of the ring carbons is preferably selected from among: 2-methylpiperidine, 2,6-dimethylpiperidine and 4-methylpiperazine.

The term "C₃-C₁₂ aryl", when it represents the R¹ and R² groups in NR¹R², means an aromatic monocyclic or polycyclic system containing between 3 and 12 atoms and optionally containing between one and three heteroatoms. Preferred compounds among the compounds of formula (1) of the present invention are those in which the C₃-C₁₂ aryl system is selected from among: phenyl, pyrrole, thiazole, pyrazole, imidazole, pyridine, pyrimidine.

The term "C₁-C₁₂ alkyl" and "substituted alkyl" means any linear or branched, saturated or unsaturated hydrocarbon chain containing between C₁-C₁₂ carbon atoms. Examples of alkyl substituents used herein are: -CH₃, -CH₂-CH₃, -CH₂-CH₂-CH₃, -CH(CH₃)₂, -C(CH₃)₃, -(CH₂)₃-CH₃, -CH₂-CH(CH₃)₂, -CH(CH₃)-CH₂-CH₃ and - CH=CH₂.

The term "cycloalkyl" means any saturated or unsaturated, monocyclic or polycyclic hydrocarbon system containing between C₃-C₁₂ carbon atoms. Examples of cycloalkyl and substituted cycloalkyl used herein are: cyclohexyl, cyclopentyl, 4-methylcyclohexyl, 4-methoxycyclohexyl, 4-carboxycyclohexyl.

The term "heteroatom" means oxygen, nitrogen or sulfur.

The term "halogen" means a substituent selected from among fluorine, chlorine, bromine and iodine.

The compounds of the formula (2) have the following structure: 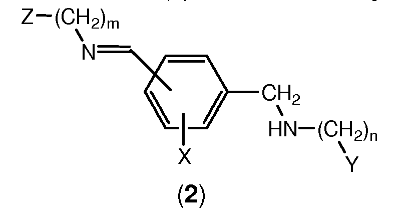 wherein:
the substituent -CH=NH-(CH₂)ₘ-Z is *meta* or *para* to the substituent -CH₂-NH-(CH₂)ₙ-Y wherein m and n, which may be identical or different, may have the values 0, 2, 3, 4, 5 and 6;
Z and Y, which may be identical or different, represent a nitrogenated heterocyclic system bonded by nitrogen (n being greater than or equal to 2) or by one of the ring carbons; a substituted nitrogenated heterocyclic system bonded by nitrogen (n being greater than or equal to 2) or by one of the ring carbons; an NR¹R² group (n being greater than or equal to 2) wherein R¹ and R² are independently selected from hydrogen, C₁-C₁₂ alkyl, substituted alkyl, C₃-C₁₂ aryl, substituted C₃-C₁₂ aryl, cycloalkyl and substituted cycloalkyl X is selected from the group consisting of hydrogen, C₁-C₁₂ alkyl, substituted alkyl, C₃-C₁₂ aryl, amino, alkylamino, nitro, hydroxy, alkoxy, halogen, carboxy or carboxamido;
and pharmaceutically acceptable salts, hydrates, solvates, esters and metal complexes of said structure.

The compounds of formula (2) can be used as precursors of the compounds of formula (1).

The compounds of the formula (3) have the following structure: 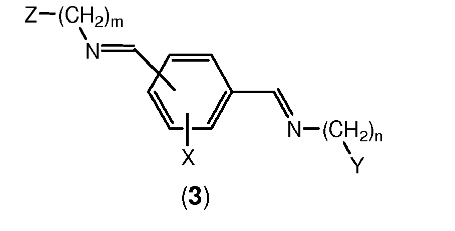 wherein:
the substituent -CH=NH-(CH₂)ₘ-Z is *meta* or *para* to the substituent -CH=NH-(CH₂)ₙ-Y wherein m and n, which may be identical or different, may have the values 0, 2, 3, 4, 5 and 6;
Z and Y, which may be identical or different, represent a nitrogenated heterocyclic system bonded by nitrogen or by one of the ring carbons; a substituted nitrogenated heterocyclic system bonded by nitrogen or by one of the ring carbons; an NR¹R² group wherein R¹ and R² are independently selected from hydrogen, C₁-C₁₂ alkyl, substituted alkyl, C₃-C₁₂ aryl, substituted C₃-C₁₂ aryl, cycloalkyl and substituted cycloalkyl
X is selected from the group consisting of hydrogen, C₁-C₁₂ alkyl, substituted alkyl, C₃-C₁₂ aryl, amino, alkylamino, nitro, hydroxy, alkoxy, halogen, carboxy or carboxamido;
and pharmaceutically acceptable salts, hydrates, solvates, esters and metal complexes of said structure.

The compounds of formula (3) can be used as precursors of the compounds of formula (1).

Preferred compounds of formula (1) useful in the present invention are selected from the group including:
*N*-(4-((2-(pyrrolidin-1-yl)ethylamino)methyl)benzyl)-2-(pyrrolidin-1-yl)ethylamine
*N*-(4-((3-(pyrrolidin-1-yl)propylamino)methyl)benzyl)-3-(pyrrolidin-1-yl)propan-1-amine
*N*-(4-((3-(1*H*-imidazol-1-yl)propylamino)methyl)benzyl)-3-(1*H*-imidazol-1-yl)propan-1-amine
*N*-(4-((2-(piperidin-1-yl)ethylamino)methyl)benzyl)-2-(piperidin-1-yl)ethylamine
*N*-(4-((3-(2-methylpiperidin-1-yl)propylamino)methyl)benzyl)-3-(2-methylpiperidin-1 - yl)propan-1-amine
*N*-(4-((3-(4-methylpiperazin-1-yl)propylamino)methyl)benzyl)-3-(4-methylpiperazin-1-yl)propan-1-amine
*N*-(4-((3-morpholinopropylamino)methyl)benzyl)-3-morpholinopropan-1-amine
*N*-(4-((2-(pyrrolidin-1-yl)ethylamino)methyl)benzyl)-3-(pyrrolidin-1-yl)propan-1-amine
*N*-(4-((2-(pyrrolidin-1-yl)ethylamino)methyl)benzyl)-3-(1*H*-imidazol-1-yl)propan-1-amine
*N*-(4-((2-(pyrrolidin-1-yl)ethylamino)methyl)benzyl)-3-(4-methylpiperazin-1-yl)propan-1-amine
*N*-(4-((2-(pyrrolidin-1-yl)ethylamino)methyl)benzyl)-3-morpholinopropan-1-amine
*N*-(4-((3-(1*H*-imidazol-1-yl)propylamino)methyl)benzyl)-3-(pyrrolidin-1-yl)propan-1-amine
*N*-(4-((2-(piperidin-1-yl)ethylamino)methyl)benzyl)-3-(pyrrolidin-1-yl)propan-1-amine
*N*-(4-((3-(pyrrolidin-1-yl)propylamino)methyl)benzyl)-3-(2-methylpiperidin-1-yl)propan-1-amine
*N*-(4-((3-(4-methylpiperazin-1-yl)propylamino)methyl)benzyl)-3-(pyrrolidin-1-yl)propan-1-amine
*N*-(4-((2-morpholinoethylamino)methyl)benzyl)-3-(pyrrolidin-1-yl)propan-1-amine
*N*-(4-((3-(pyrrolidin-1-yl)propylamino)methyl)benzyl)-3-morpholinopropan-1-amine
*N*-(4-((2-(piperidin-1-yl)ethylamino)methyl)benzyl)-3-(1*H*-imidazol-1-yl)propan-1 - amine
*N*-(4-((3-(1*H*-imidazol-1-yl)propylamino)methyl)benzyl)-3-(2-methylpiperidin-1-yl)propan-1-amine
*N*-(4-((3-(4-methylpiperazln-1-yl)propylamino)methyl)benzyl)-3-(1*H*-imidazol-1-yl)propan-1-amine
*N*-(4-((2-morpholinoethylamino)methyl)benzyl)-3-(1*H*-imidazol-1-yl)propan-1-amine
*N*-(4-((3-morpholinopropylamino)methyl)benzyl)-3-(1*H*-imidazol-1-yl)propan-1-amine
*N*-(4-((2-(piperidin-1-yl)ethylamino)methyl)benzyl)-3-(4-methylpiperazin-1-yl)propan-1-amine
*N*-(4-((2-(piperidin-1-yl)ethylamino)methyl)benzyl)-3-morpholinopropan-1-amine
*N*-(4-((3-(4-methylpiperazin-1-yl)propylamino)methyl)benzyl)-3-(2-methylpiperidin-1-yl)propan-1-amine
*N*-(4-((2-morpholinoethylamino)methyl)benzyl)-3-(2-methylpiperidin-1-yl)propan-1-amine
*N*-(4-((3-morpholinopropylamino)methyl)benzyl)-3-(2-methylpiperidin-1-yl)propan-1-amine
*N*-(4-((2-morpholinoethylamino)methyl)benzyl)-3-(4-methylpiperazin-1-yl)propan-1-amine
*N*-(4-((3-morpholinopropylamino)methyl)benzyl)-3-(4-methylpiperazin-1-yl)propan-1-amine
*N*-(4-((2-morpholinoethylamino)methyl)benzyl)-3-morpholinopropan-1-amine

Preferred compounds of formula (2) useful in the present invention are selected from the group including:
*N*-(4-((2,6-dimethylpiperidin-1-ylimino)methyl)benzyl)-2-(pyrrolidin-1-yl)ethylamine
*N*-(4-((2,6-dimethylpiperidin-1-ylimino)methyl)benzyl)-3-(1*H*-imidazol-1-yl)propan-1-amine
*N*-(4-((2,6-dimethylpiperidin-1-ylimino)methyl)benzyl)-3-(4-methylpiperazin-1-yl)propan-1-amine
*N*-(4-((2,6-dimethylpiperidin-1-ylimino)methyl)benzyl)-3-morpholinopropan-1-amine
*N*-(4-((4-methylpiperazin-1-ylimino)methyl)benzyl)-2-(pyrrolidin-1-yl)ethanamine
*N*-(4-((piperidin-1-ylimino)methyl)benzyl)-3-(pyrrolidin-1-yl)propan-1-amine
*N*-(4-((piperidin-1-ylimino)methyl)benzyl)-3-(1*H*-imidazol-1-yl)propan-1-amine
*N*-(4-((piperidin-1-ylimino)methyl)benzyl)-3-(2-methylpiperidin-1-yl)propan-1-amine
*N*-(4-((piperidin-1-ylimino)methyl)benzyl)-3-(4-methylpiperazin-1-yl)propan-1-amine
*N*-(4-((piperidin-1-ylimino)methyl)benzyl)-3-morpholinopropan-1-amine
*N*-(4-((2,6-dimethylpiperidin-1-ylimino)methyl)benzyl)-3-(pyrrolidin-1-yl)propan-1-amine
*N*-(4-((4-methylpiperazin-1-ylimino)methyl)benzyl)-3-(pyrrolidin-1-yl)propan-1-amine
*N*-(4-((4-methylpiperazin-1-ylimino)methyl)benzyl)-3-(1*H*-imidazol-1-yl)propan-1-amine
*N*-(4-((4-methylpiperazin-1-ylimino)methyl)benzyl)-3-(2-methylpiperidin-1-yl)propan-1-amine
*N*-(4-((4-methylpiperazin-1-ylimino)methyl)benzyl)-3-(4-methylpiperazin-1-yl)propan-1-amine
*N*-(4-((4-methylpiperazin-1-ylimino)methyl)benzyl)-3-morpholinopropan-1amine

Preferred compounds of formula (3) useful in the present invention are selected from the group including:
((4-(N-(piperidin-1-yl)imino)methyl)phenyl)-N-(piperidin-1-yl)methanimine
((4-(N-(2,6-dimethylpiperidin-1-yl)imino)methyl)phenyl)-N-(2,6-dimethylpiperidin-1-yl) methanimine
((4-(N-(4-methylpiperazin-1-yl)imino)methyl)phenyl)-N-(piperidin-1-yl)methanimine

The compounds of formulae (1), (2) and (3) are prepared as shown in Schemes 1 to 3, wherein the variables Z, Y, n, m, and X are selected such that the corresponding substituents do not include any combination which renders the processes of Schemes 1 to 3 inoperative. All the starting products are commercially available or can be obtained from commercially available products by skilled personnel.

The compounds of general formula (1) can be prepared according to Scheme 1:

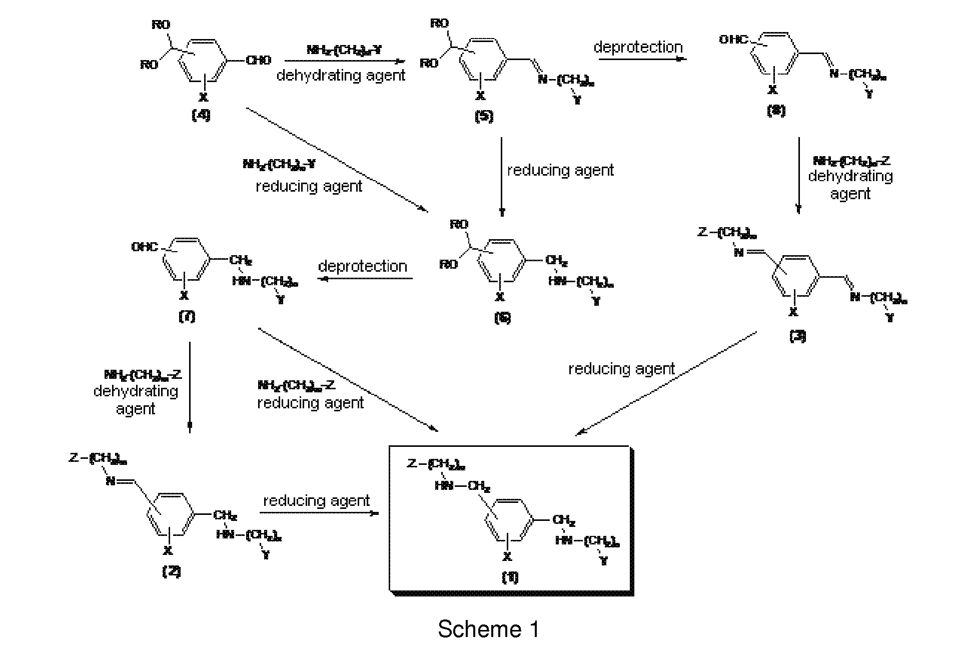

The reductive amination of a dialkoxymethylbenzaldehyde of general structure (4), wherein the term "alkoxy" means -Oalkyl such as the alkyl which has been defined previously, with an amino derivative of general structure NH₂-(CH₂)ₙ-Y (n ≥ 2 if the system Y is bonded by nitrogen) in the presence of a reducing agent, preferably sodium borohydride or sodium cyanoborohydride, to yield, through the monoimine (5), the intermediate acetal of formula (6). Alternatively, it is possible to isolate the monoimine (5) upon treating the compound (4) with the amine NH₂-(CH₂)ₙ-Y in the presence of a dehydrating agent, preferably molecular sieves. The deprotection of the acetal of (6), for example in aqueous acid medium with 2 M hydrochloric acid, yields the aldehyde (7). The treatment of (7) with the corresponding amino derivative of general formula NH₂-(CH₂)ₘ-Z (m ≥ 2 if the system Z is bonded by nitrogen) in the presence of a reducing agent, preferably sodium borohydride or sodium cyanoborohydride, leads to the final symmetrical (if Z = Y and m = n) or asymmetric diamine system (1). Alternatively, it is possible to obtain by treatment of (7) with the corresponding amino derivative NH₂-(CH₂)ₘ-Z, in the presence of a dehydrating agent, the monoimines (2), compounds which are also object of the present invention, which, if desired, can subsequently be reduced to the corresponding compound of formula (1). It is also possible to obtain the aldehyde (8) starting from the monoimine (5), by the deprotection of the acetal group, which aldehyde treated with an amino derivative of general structure NH₂-(CH₂)ₘ-Z in the presence of a dehydrating agent, preferably molecular sieves, yields the diimines (3), compounds which are also object the present invention, which, if desired, can subsequently be reduced to the corresponding compound of formula (1).

In the specific case of compounds (1a), (1) wherein X = H and the substituents -CH₂-NH-(CH₂)ₘ-Z and -CH₂-NH-(CH₂)ₙ-Y are *para* to one another, the starting compound 4 could correspond to the commercial product 4-(diethoxymethyl)benzaldehyde (4a).

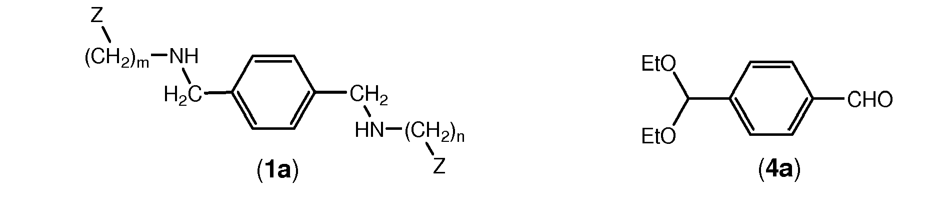

The compounds of formula (1b), (1) wherein Y = Z and m = n, can be prepared by methods similar to those described, in Scheme 1 or in Scheme 2.

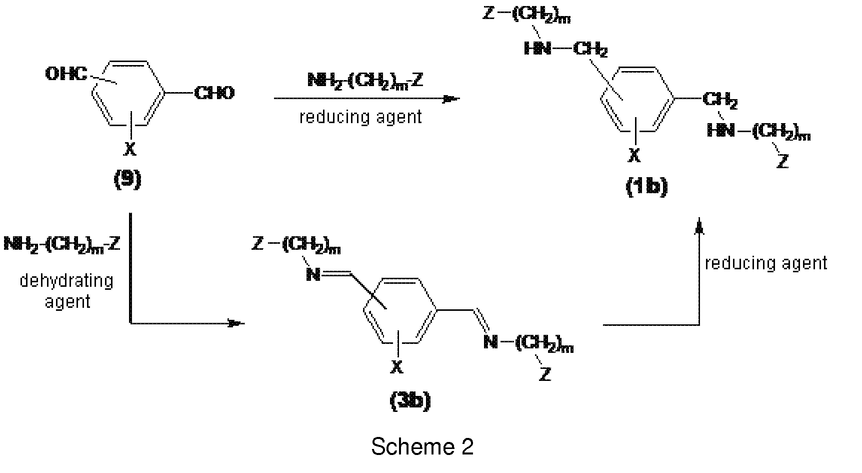

Thus, the treatment of a dialdehyde of general structure (9) with an amino derivative of general structure NH₂-(CH₂)ₘ-Z in the presence of a reducing agent, preferably sodium borohydride or sodium cyanoborohydride, yields a symmetrical compound of formula (1b). Said compound (1b) is also accessible by treatment of (9) with an amino derivative of general structure NH₂-(CH₂)ₘ-Z in the presence of a dehydrating agent, preferably molecular sieves, to yield the symmetrical diimine (3b), which is reduced to the diamine (1b) with the same type of reducing agent.

In the specific case of compounds (1c), (1) wherein X=H, Y=Z, m=n and the substituents -CH₂-NH-(CH₂)ₘ-Z are *para* to one another, the starting compound (9) could correspond to the commercial product terephthaldehyde (9a).

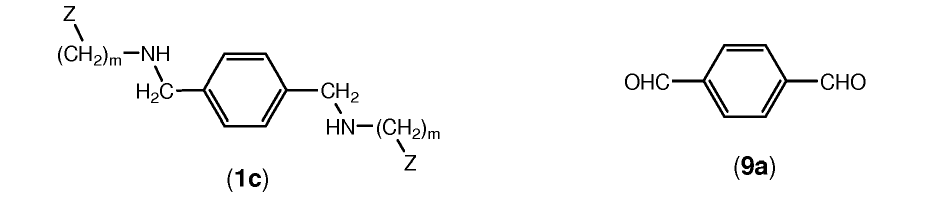

The compounds of general formula (2), which can also be obtained according to Scheme 1, are also an object of the present invention. In the specific case of compounds (2a), (2) wherein X=H and the substituents -CH₂-NH-(CH₂)ₘ-Z and -CH₂-NH-(CH₂)ₙ-Y are *para* to one another, the starting compound (4) could correspond to the commercial product 4-(diethoxymethyl)benzaldehyde (4a).

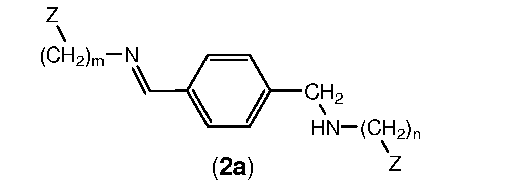

The compounds of general formula (3), which can also be obtained according to Scheme 1, are also an object of the present invention. In the specific case of compounds (3b), (3) wherein m = n and Z = Y, these compounds can be obtained according to Scheme 1 or Scheme 2.

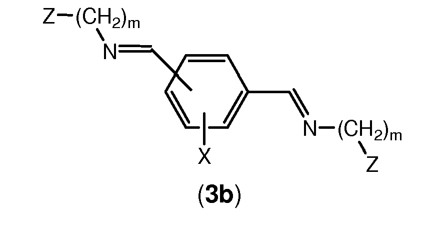

In the specific case of compounds (3c), (3) wherein X = H, Y = Z, m = n and the substituents -CH₂-NH-(CH₂)ₘ-Z are *para* to one another, the starting compound (9) could correspond to the commercial product terephthaldehyde (9a).

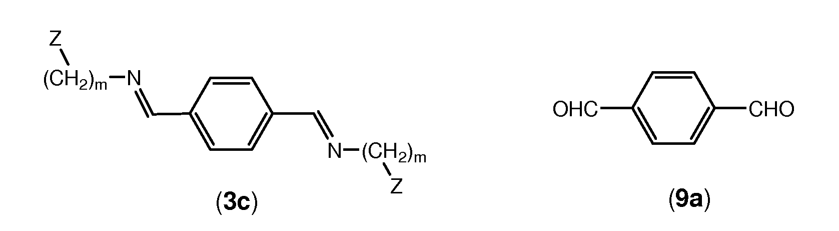

In the specific case of compounds (3d), (3) wherein n = 0 and Y is bonded by nitrogen, these compounds can also be obtained according to Scheme 3 by treatment of a dialdehyde (9) with a hydrazine NH₂-Y in the presence of a dehydrating agent to yield the aldehyde (10). The treatment of (10) with an amino derivative of formula NH₂-(CH₂)ₘ-Z in the presence of a dehydrating agent yields (3d).

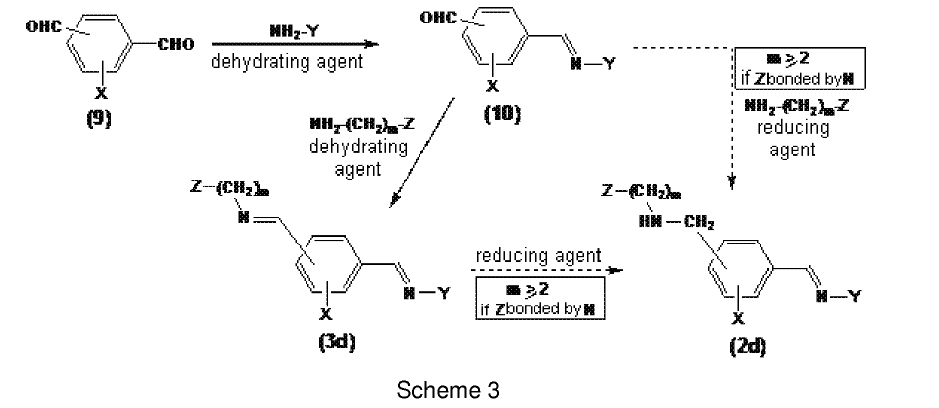

In the specific case of compounds (3d) in which the system NH₂-(CH₂)ₘ-Z used has not been a hydrazine (m ≥ 2 if Z is bonded by nitrogen), the subsequent treatment with a reducing agent yields compounds (2d). It should be emphasized that (2d) can be obtained from (10) *in situ* without isolating (3d) by treatment with the amino derivative of general structure NH₂-(CH₂)ₘ-Z in the presence of a reducing agent.

In the specific case of compounds (3e), (3) wherein n = 0, X = H and the substituents are *para* to one another, the starting compound (9) would correspond to terephthaldehyde (9a).

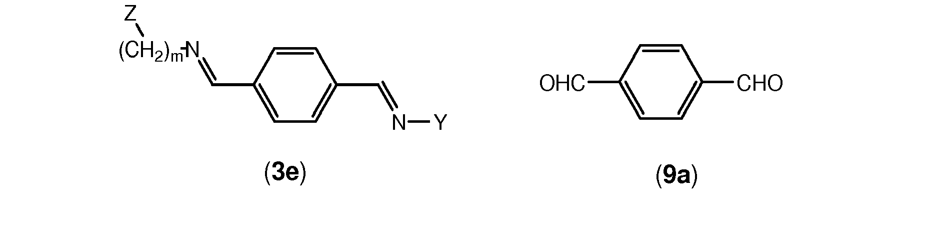

Finally, the compounds (1), (2) and (3) in which X represents hydrogen and both substituents are *meta* to one another, can be prepared by skilled personnel from isophthaldehyde (11) and from 3-(diethoxymethyl)benzaldehyde (12), both compounds being commercially available, by means of processes similar to those described in Schemes 1 to 3.

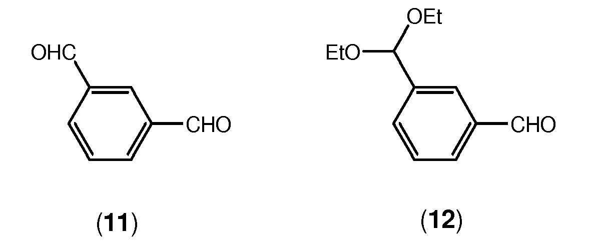

The pharmaceutically acceptable salts, hydrates, solvates, esters and metal complexes of the compounds of formula **(1), (2)** and **(3)** object of the present invention can be obtained from skilled personnel from commercially available starting products.

The biological activity as anti-HIV agents of the compounds of formula (1), (2) and (3) object of the present invention has been demonstrated by means of the following *in vitro* test.

### Protocol for evaluating the anti-HIV activity of compounds in MT-4 cells.

The assay for evaluating the anti-HIV activity of compounds is based on determining cell viability by the methyl-thiazole-tetrazolium (MTT) reduction method. The *in vitro* assays consist of culturing for MT-4 lymphoid cells for 5 days in the presence of serial dilutions of the compounds to be tested in the presence or absence of virus. The culture of cells and compound alone allows determining the CC₅₀ or cytotoxic concentration 50, the concentration at which the compound induces death in 50 % of the cell culture. The culture of the cells and compound in the presence of virus allows evaluating the EC₅₀ or effective concentration 50, the concentration at which the compound inhibits 50% of the cytopathic effect induced by HIV. The anti-HIV activity of compounds with known activity is evaluated in each assay to validate the assay.

The compounds of formula (1), (2) and (3) object of the present invention generally have an EC₅₀ < 10 µg/mL. Three of the assayed compounds have shown an EC₅₀ < 0.05 µg/mL. The CC₅₀ values are generally greater than 25 µg/mL.

Without going into further detail, it is considered that a skilled person can, using the previous description, use the present invention in its entire depth. The following examples are set forth below in order to better understand the invention, but must not be considered as limitations thereto:

The amino derivatives of general formulae NH₂-(CH₂)ₙ-Y and NH₂-(CH₂)ₘ-Z used are shown in Figure 1. The numbering of the compounds (1), (2) and (3) follows the format 1 {*amino derivative Z, amino derivative Y*}.

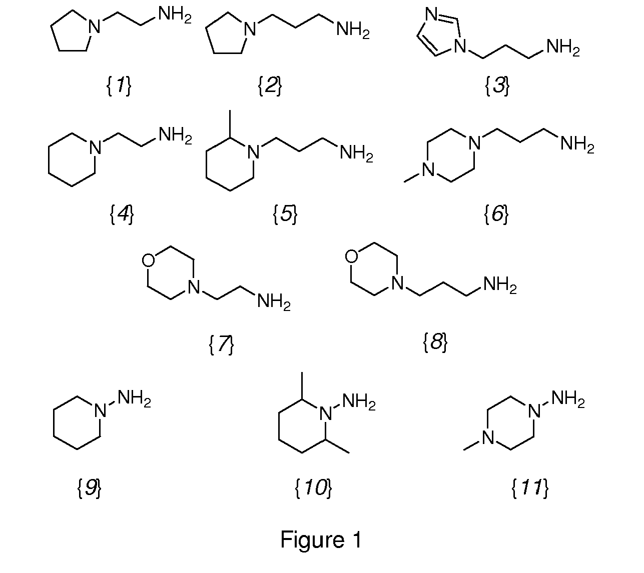

### Obtaining N-(4-((2-(Pyrrolidin-1-yl)ethylamino)methyl)benzyl)-2-(Pyrrolidin-1-yl)ethylamine (1{1,1); X=H, m=n=2, Z=Y=pyrrolidin-1-yl):

0.61 g (4.5 mmol) of terephthaldehyde (9a) and 1.04 g (9.0 mmol) of 2-(pyrrolidin-1-yl)ethylamine 5{*1*} (m=2, Z=pyrrolidin-1-yl) are dissolved in 30 mL of anhydrous MeOH. Molecular sieve (4Å) is added and it is stirred at reflux temperature under a nitrogen atmosphere for 24 h. The molecular sieve is filtered and 0.34 g (9.0 mmol) of NaBH₄ are added. It is allowed to react at room temperature for 16 h. After this time water is added and it is extracted with CH₂Cl₂. The organic phase is washed with brine and dried on anhydrous MgSO₄. The solvent is eliminated under reduced pressure and 1.32 g (4.0 mmol, 89%) of a yellow oil 1{*1,1*} are obtained. IR (film): ν (cm⁻¹) 3310, 2962, 2928, 2874, 2794, 1485, 1444, 775.¹H-NMR (300 MHz, CDCl₃): δ (ppm) 7.27 (s, 4H, Ph), 3.79 (s, 4H, CH₂-Ph), 2.73 (t, 4H, *J*=6.0 Hz, CH₂-N), 2.59 (t, 4H, *J*=6.0 Hz, CH₂-N), 2.47 (m, 8H, CH₂-N), 2.20 (bs, 2H, NH), 1.75 (m, 8H, *J*=3.3 Hz, CH₂). ¹³C-NMR (75 MHz, CDCl₃): δ (ppm) 138.8, 128.0, 55.9, 54.2, 53.8, 47.8, 23.5. MS (FAB): m/z 331.3 [M+1H]⁺, 330.3, 84.0. HRMS: Calculated for C₂₀H₃₅N₄: 331.2862 [M+1H]⁺. Obtained: 331.2867.

### Obtaining N-(4-((3-(Pyrrolidin-1-yl)propylamino)methyl)benzyl)-3-(pyrrolidin-1-yl)propan-1-amine (1{2,2}; X=H, m=n=3, Z=pyrrolidin-1-yl):

As for 1{*1,1*}, but using 0.75 g (5.5 mmol) of terephthaldehyde (9a), 1.47 g (11.1 mmol) of 3-(pyrrolidin-1-yl)propan-1-amine {*2*} and 0.43 g (11.1 mmol) of NaBH₄. 1.98 g (5.5 mmol, 99%) of a yellow oil 1{*2,2*} are obtained. IR (CHCl₃ evaporated film): ν (cm⁻¹) 3282, 2936, 2789, 1458. ¹H-NMR (300 MHz, CDCl₃): δ (ppm) 7.26 (s, 4H, Ph), 3.77 (s, 4H, CH₂-Ph), 2.69 (t, 4H, *J*=7.0 Hz, CH₂-N), 2.49 (m, 12H, CH₂-N), 2.08 (bs, 2H, NH), 1.75 (m, 12H, CH₂). ¹³C-NMR (75 MHz, CDCl₃): δ (ppm) 138.5, 128.0, 54.6, 54.1, 53.5, 47.9, 28.8, 23.4. MS (IE): m/z 359.2 [M+1H]⁺, 231.0, 230.0, 127.0, 98.0, 84.0. HRMS: Calculated for C₂₂H₃₉N₄: 359.3175. Obtained: 359.3169.

### Obtaining N-(4-((3-(1H-imidazol-1-yl)propylamino)methyl)benzyl)-3-(1H-imidazol-1-yl)propan-1-amine (1{3,3}; X=H, m=n=3, Z=1H-imidazol-1-yl):

As for 1{*1,1*}, but using 0.54 g (3.9 mmol) of terephthaldehyde (9a), 1.00 g (7.8 mmol) of 3-(1*H*-imidazol-1-yl)propan-1-amine {*3*} and 0.30 g (7.8 mmol) of NaBH₄. 1.38 g (3.9 mmol, 100%) of a yellow oil 1{*3,3*} are obtained. IR (CHCl₃ evaporated film): ν (cm⁻¹) 3277, 3103, 2935, 2815, 1508, 1453. ¹H-NMR (300 MHz, CDCl₃): δ (ppm) 7.41 (s, 2H, N=CH), 7.26 (s, 4H, Ph), 7.02 (s, 2H, CH), 6.88 (s, 2H, CH), 4.04 (t, 4H, *J*=6.9 Hz, CH₂-N), 3.74 (s, 4H, CH₂-Ph), 2.60 (t, 4H, *J*=6.9 Hz, CH₂-N), 2.05 (s, 2H, NH), 1.92 (m, 4H, 6.9 Hz, CH₂). ¹³C-NMR (75 MHz, CDCl₃): δ (ppm) 138.8, 137.1, 129.2, 128.2, 118.7, 53.6, 45.6, 44.6, 31.3. MS (IE): m/z 353.0 [M+1H]⁺, 260.0, 228.9, 138.0, 81.0. HRMS: Calculated for C₂₀H₂₈N₆: 352.2454 [M+1H]⁺. Obtained: 353.2448.

### Obtaining N-(4-((2-(Piperidin-1-yl)ethylamino)methyl)benzyl)-2-(piperidin-1-yl)ethylamine (1{4,4}; X=H, m=n=2, Z= piperidin-1-yl):

As for 1{*1,1*}, but using 0.29 g (2.1 mmol) of terephthaldehyde (9a), 0.56 g (4.3 mmol) of 2-(piperidin-1-yl)ethylamine {*4*} and 0.16 g (4.3 mmol) of NaBH₄. 0.76 g (2.1 mmol, 100%) of a yellow oil 1{*4,4*} are obtained. IR (CHCl₃ evaporated film: ν (cm⁻¹) 3310. ¹H-NMR (300MHz, CDCl₃): δ (ppm) 7.32 (s, 4H, Ph), 3.75 (s, 4H, CH₂-Ph), 2.69 (t, *J*=7.0 Hz, 4H, CH₂-N), 2.47 (t, *J*=7.0 Hz, 4H, CH₂-N), 2.39 (m, 8H, CH₂-N), 1.62-1.39 (m, 12H, CH₂). ¹³C-NMR (75.5MHz, CDCl₃): δ (ppm) 139.4, 129.6, 55.7, 59.0, 54.1, 46.1, 26.7, 25.2. Anal. Calculated for C₂₂H₃₈N₄: C 73.69%, H 10.68%, N 15.63%. Obtained: C 73.78%, H 10.56%, N 15.45%.

### Obtaining N-(4-((3-(2-methylpiperidin-1-yl)propylamino)methyl)benzyl)-3-(2-methylpiperidin-1-yl)propan-1-amine (1{5,5}; X=H, m=n=3, Z= 2-methylpiperidin-1-yl:

As for 1{*1,1*}, but using 0.43 g (3.2 mmol) of terephthaldehyde (9a), 1.04 g (6.4 mmol) of 3-(2-methylpiperidin-1-yl)propan-1-amine {*5*} and 0.25 g (6.4 mmol) of NaBH₄. 1.33 g (3.2 mmol, 100%) of a pale brown oil 1{*5,5*} are obtained. IR (CHCl₃ evaporated film): ν (cm⁻¹) 3282, 2929, 2854, 2793, 1449, 1372. ¹H-NMR (300 MHz, CDCl₃): δ (ppm) 7.27 (s, 4H, Ph), 3.77 (s, 4H, CH₂-Ph), 2.87 (m, 2H, CH), 2.73 (m, 2H, CH₂), 2.63 (t, 4H, *J*=6.8 Hz, CH₂), 2.36 (m, 2H, CH₂), 2.26 (m, 2H, CH₂), 2.14 (bs, 2H, NH), 2.11 (m, 2H, CH₂), 1.73-1.44 (m, 12H, CH₂), 1.27 (m, 4H, CH₂), 1.05 (d, 6H, *J*=6.3 Hz, CH₃). ¹³C-NMR (75 MHz, CDCl₃): δ (ppm) 138.8, 128.0, 55.9, 53.7, 52.3, 52.1, 48.3, 34.7, 26.2, 25.7, 24.0, 19.1. MS (IE): m/z 415.4 [M+1H]⁺, 112.1. Anal. Calculated for C₂₆H₄₆N₄: C 75.31%, H 11.18%, N 13.51%. Obtained: C 75.21%, H 10.92%, N 13.48%.

### Obtaining N-(4-((3-(4-methylpiperazin-1-yl)propylamino)methyl)benzyl)-3-(4-methylpiperazin-1-yl)propan-1-amine (1{6,6} X=H, m=n=3, Z= 4-methylpiperazin-1-yl:

As for 1{*1,1*}, but using 0.48 g (3.5 mmol) of terephthaldehyde (9a), 1.14 g (7.0 mmol) of 3-(4-methylpiperazin-1-yl)propan-1-amine {*6*} and 0.27 g (7.0 mmol) of NaBH₄. 1.28 g (3.1 mmol, 87%) of a brown oil 1{*6,6*} are obtained. IR (CHCl₃ evaporated film): ν (cm⁻¹) 3281, 2935, 2793, 1458. ¹H-NMR (300 MHz, CDCl₃): δ (ppm) 7.26 (s, 4H, Ph), 3.76 (s, 4H, CH₂-Ph), 2.66 (t, 4H, *J*=6.9 Hz, CH₂-N), 2.42 (m, 22H, CH₂, NH), 2.27 (s, 6H, CH₃), 1.70 (m, 4H, 6.9 Hz, CH₂). ¹³C-NMR (75 MHz, CDCl₃): δ(ppm) 138.8, 128.0, 57.0, 55.1, 53.7, 53.2, 48.1, 46.0, 26.9. MS (IE): m/z 417.0 [M+1H]⁺, 416.0, 289.0, 260.0, 156.0, 141.0, 127.0, 113.0. HRMS: Calculated for C₂₄H₄₄N₆: 417.3706 [M+1H]⁺. Obtained: 417.3700.

### Obtaining N-(4-((2-morpholinoethylamino)methyl)benzyl)-2-morpholinoethylamine (1{7,7}; X=H, m=n=2, Z= morpholino):

Like 1{*1,1*} but using 0.54 g (4.0 mmol) of terephthaldehyde (9a), 1.05 g (8.0 mmol) of 2-morpholinoethylamine {*7*} and 0.31 g (8.0 mmol) of NaBH₄. 0.93 g (2.6 mmol, 64%) of an off-white solid 1{*7,7*} are obtained. IR (CHCl₃ evaporated film): ν (cm⁻¹) 3341 2968, 2930, 2817, 1446, 1117. ¹H-NMR (300 MHz, CDCl₃): δ (ppm) 7.27 (s, 4H, Ph), 3.79 (s, 4H, CH₂-Ph), 3.69 (t, 8H, *J*=4.6 Hz, CH₂-O), 2.70 (t, 4H, *J*=6.0 Hz, CH₂), 2.496 (t, 4H, *J*=6.0 Hz, CH₂), 2.403 (t, 8H, *J*=4.6 Hz, CH₂-N), 1.82 (s, 2H, NH). ¹³C-NMR (75 MHz, CDCl₃): δ (ppm) 139.0, 128.0, 67.0, 58.3, 53.7, 53.7, 45.3. MS (IE): m/z 363.4 [M+1H]⁺, 362.4, 262.3, 233.2, 232.2, 100.0. Anal. Calculated for C₂₀H₃₄N₄O₂: C 66.26%, H 9.45%, N 15.46%, O 8.83%. Obtained: C 66.40%, H 9.68%, N 15.43%.

### Obtaining N-(4-((3-morpholinopropylamino)methyl)benzyl)-3-morpholinopropan-1-amine (1{8,8}; X=H, m=n=3, Z= morpholino):

As for 1{*1,1*}, but using 0.47 g (3.5 mmol) of terephthaldehyde (9a), 1.00 g (7.0 mmol) of 3-morpholinopropan-1-amine {*8*} and 0.27 g (7.0 mmol) of NaBH₄. 1.16 g (3.0 mmol, 85%) of a yellow oil 1{*8,8*} are obtained. IR (CHCl₃ evaporated film): ν (cm⁻¹) 3301, 2948, 2852, 2806, 1456, 1118. ¹H-NMR (300 MHz, CDCl₃): δ (ppm) 7.27 (s, 4H, Ph 3.79 (s, 4H, CH₂-Ph 3.70 (t, 8H, *J*=6 Hz, CH₂O), 2.68 (t, 4H, *J*=7.0 Hz, CH₂-NH), 2.41 (m, 12H, CH₂-N), 1.81 (s, 2H, NH), 1.70 (m 4H, *J*=7.0 Hz, CH₂). ¹³C-NMR (75 MHz, CDCl₃): δ (ppm) 138.9, 128.0, 67.0, 57.39, 53.8, 53.8, 48.0, 26.8. MS (IE): m/z 390.3 [M]⁺, 247.2, 100.0. HRMS: Calculated for C₂₂H₃₈N₄O₂: 390.2995. Obtained: 390.2995.

### Obtaining ((4-(N-(piperidin-1-yl)imino)methyl)phenyl)-N-(piperidin-1-yl)methanimine (3{9,9}; X=H, m=n=0, Z=Y=piperidin-1-yl):

1.51 g (14.7 mmol) of 1-aminopiperidine {*9*} are dissolved in 30 mL of anhydrous MeOH. Immediately afterwards, 0.992g (7.33mmol) of terephthaldehyde (9a) are added. The resulting solution is heated under reflux for 16h under a nitrogen atmosphere and in the presence of 4Å molecular sieve. The molecular sieve is then filtered in hot conditions. The filtrate is collected and the solvent is partially eliminated almost to dryness and is cooled at 4°C. The solid is filtered and washed with cold MeOH. The obtained solid is dried on P₂O₅. 1.42 g (4.8 mmol, 65%) of a yellow solid 3{*9,9*} are obtained. IR (CHCl₃ evaporated film): ν (cm⁻¹) 1576. ¹H-NMR (300 MHz, CDCl₃): δ (ppm) 7.55 (s, 4H, Ph), 7.53 (s, 2H, CH=N), 3.16 (m, 8H, CH₂-N), 1.79-1.71 (m, 8H, CH₂), 1.58-1.50 (m, 4H, CH₂). ¹³C-NMR (75 MHz, CDCl₃): δ (ppm) 136.0, 134.3, 125.9, 52.1, 25.3, 24.2. Anal. Calculated for C₁₈H₂₆N₄: C 72.44%, H 8.78%, N 18.77%. Obtained: C 72.40%, H 8.81%, N 18.83%.

### Obtaining ((4-(N-(2,6-dimethylpiperidin-1-yl)imino)methyl)phenyl)-N-(2,6-dimethylpiperidin-1-yl)methanimine (3{10,10}; X=H, m=n=0, Z=Y=2,6-dimethylpiperidin-1-yl):

As for 3{*9,9*} but using 1.20 g (8.4 mmol) of 1-amino-2,6-dimethylpiperidine {*10*} and 0.57 g (4.2 mmol) of terephthaldehyde (9a). 1.05 g (3.0 mmol, 65%) of a yellow solid 3{*10,10*} are obtained. IR (CHCl₃ evaporated film): ν (cm⁻¹) 1620. ¹H-NMR (300MHz, CDCl₃): δ (ppm) 7.87 (s, 2H, CH=N), 7.64 (s, 4H, Ph), 3.31-3.27 (m, 4H, CH), 1.83-1.51 (m, 12H, CH₂), 1.03 (d, *J*=6.0 Hz, 12H, CH₃). ¹³C-NMR (75.5MHz, CDCl₃): δ (ppm) 146.6, 136.0, 126.8, 56.1, 32.4, 20.0, 19.9. Anal. Calculated for C₂₂H₃₄N₄ : C 74.53%, H 9.67%, N 15.80%. Obtained: C 74.30%, H 9.35%, N 15.84%.

### Obtaining ((4-(N-(4-methylpiperazin-1-yl)imino)methyl)phenyl)-N-(4-methylpiperazin-1-yl)methanimine (3{11,11}; X=H, m=n=0, Z=Y=4-methylpiperazin-1-yl):

As for 3{*9,9*}, but using 0.64 g (5.4 mmol) of 1-amino-4-methylpiperazine {*11*} and 0.37 g (2.7 mmol) of terephthaldehyde (9a). 0.67 g (2.0 mmol, 75%) of a yellowish solid 3{*11,11*} are obtained. IR (CHCl₃ evaporated film): ν (cm⁻¹) 1579. ¹H-NMR (300MHz, CDCl₃): δ (ppm) 7.57 (s, 4H, Ph), 7.53 (s, 2H, CH=N), 3.23 (m, 8H, CH₂-N), 2.62 (m, 8H, CH₂-N), 2.36 (s, 6H, CH₃). ¹³C-NMR (75.5MHz, CDCl₃): δ (ppm) 135.8, 135.4, 126.2, 54.5, 51.0, 46.0. Anal. Calculated for C₁₈H₂₈N₆: C 65.82%, H 8.59%, N 25.59%. Obtained: C 65.51%, H 8.72%, N 25.17%.

### Obtaining 4-((2-(pyrrolidin-1-yl)ethylamino)methyl)benzaldehyde (7{1}; X=H, m=2, Z= pyrrolidin-1-yl):

2.01 g (9.3 mmol) of diethyl monoacetal of the terephthaldehyde (4a) and 1.09 g (9.3 mmol) of 2-(pyrrolidin-1-yl)ethylamine {*1*} are dissolved in 30 mL of anhydrous MeOH. 4Å molecular sieve is added and it is stirred at reflux temperature and under a nitrogen atmosphere for 36 h. The molecular sieve is filtered and 0.36 g (9.3 mmol) of NaBH₄ are added. It is allowed to react at room temperature for 5 h. Water is added and it is extracted with CH₂Cl₂. The organic phase is washed with brine and dried on anhydrous MgSO₄. The solvent is eliminated under reduced pressure and 2.66 g (8.7 mmol, 93%) of a yellow oil corresponding to the acetal 8{*1*} are obtained. 20 mL of 2M HCl are added to 2.64 g (8.6 mmol) of the acetal 8{*1*} and it is stirred at room temperature for 2 h. It is basified with NaOH and extracted with CH₂Cl₂. The organic phase is washed with brine and dried on MgSO₄. The solvent is eliminated under reduced pressure and 1.79 g (7.7 mmol, 89% yield) of (7{*1*} are obtained.IR (film): ν (cm⁻¹) 3309, 3051, 2961, 2930, 2875, 2799, 1700, 1606, 780. ¹H-NMR (300 MHz, CDCl₃): δ (ppm) 10.00 (s, 1H, CHO), 7.84 (d, 2H, 8.1 Hz, Ph), 7.51 (d, 2H, *J*=8.1 Hz, Ph), 3.90 (s, 2H, CH₂-Ph), 2.75 (t, 2H, *J*=6.0 Hz, CH₂-N), 2.64 (t, 2H, *J*=6.0 Hz, CH₂-N), 2.51 (m, 4H, CH₂-N), 2.01 (bs, 1 H, NH), 1.77 (m, 4H, CH₂). ¹³C-NMR (75 MHz, CDCl₃): δ (ppm) 191.8, 147.6, 135.1, 129.7, 128.4, 55.8, 54.2, 53.7, 47.8, 23.5. MS (IE): m/z 233.2 [M+ 1H]⁺, 232.2, 148.1, 119.0, 84.1. HRMS: Calculated for C₁₄H₂₀N₂O: 232.1576. Obtained: 232.1572.

### Obtaining 4-((3-(pyrrolidin-1-yl)propylamino)methyl)benzaldehyde (7{2}; X=H, m=3, Z= pyrrolidin-1-yl):

As for 7{*1*}, but using 6.01 g (28.0 mmol) of diethyl monoacetal of the terephthaldehyde (4a), 3.70 g (28.0 mmol) of 3-(pyrrolidin-1-yl)propan-1-amine {*2*} and 1.07 g (28.0 mmol) of NaBH₄. 8.01 g (25.0 mmol, 89%) of a yellow oil corresponding to the acetal 8{*2*} are obtained. 7.98 g (24.9 mmol) of 8{*2*} are hydrolized with 2M HCl and 4.59 g (18.6 mmol, 75%) of 7{*2*} are obtained. IR (film): ν (cm⁻¹) 3276, 2934, 2874, 2790, 1700, 1606, 1458, 822. ¹H-NMR (300 MHz, CDCl₃): δ (ppm) 10.00 (s, 1H, CHO), 7.84 (d, 2H, Ph), 7.50 (d, 2H, Ph), 3.88 (s, 2H, CH₂-Ph), 2.70 (t, 2H, CH₂-N), 2.51 (m, 6H, CH₂-N), 1.87 (bs, 1H, NH), 1.75 (m, 6H, CH₂). ¹³C-NMR (75 MHz, CDCl₃): δ (ppm) 191.8, 147.7, 135.2, 129.8, 128.3, 54.7, 54.3, 53.7, 48.2, 29.2, 23.5. MS (IE): m/z 247.2 [M+1H]⁺, 246.2, 127.2, 119.0, 84.1. Anal. Calculated for C₁₅H₂₂N₂O: C 73.13%, H 9.00%, N 11.37%, O 6.49%. Obtained: C 73.28%, H 9.30%, N 11.45%.

### Obtaining 4-((3-(1H-imidazol-1-yl)propylamino)methyl)benzaldehyde (7{3}; X=H, m=3, Z= 1H-imidazol-1-yl):

As for 7{*1*}, but using 2.01 g (9.3 mmol) of diethyl monoacetal of the terephthaldehyde (4a), 1.20 g (9.3 mmol) of 3-(1*H*-imidazol-1-yl)propan-1-amine {*3*} and 0.36 g (9.3 mmol) of NaBH₄. 2.68 g (8.4 mmol, 90%) of a yellow oil corresponding to the acetal 8{*3*} are obtained. 2.68 g (8.4 mmol) of 8{*3*} are hydrolized with 2M HCl and 1.76 g (7.2 mmol, 86%) of a yellowish oil 7{*3*} are obtained. IR (film): ν (cm⁻¹) 3268, 3108, 2936, 2831, 2738, 1696, 1606, 1508. ¹H-NMR (300 MHz, CDCl₃): δ (ppm) 10.00 (s, 1 H, CHO), 7.85 (d, 2H, *J*=8.1Hz, Ph), 7.48 (d, 2H, *J*=8.1 Hz, C_{Ar}H), 7.46 (s, 1H, CH), 7.05 (s, 1H, CH), 6.90 (s, 1H, CH), 4.07 (t, 2H, *J*=6.9 Hz, CH₂-N), 3.85 (s, 2H, CH₂-Ph), 2.62 (t, 2H, *J*=6.9 Hz, CH₂-N), 1.95 (m, 2H, *J*=6.9 Hz, CH₂), 1.75 (bs, 1 H, NH). ¹³C-NMR (75 MHz, CDCl₃): δ (ppm) 191.7, 147.2, 137.0, 135.3, 129.8, 129.3, 128.4, 118.7, 53.6, 45.8, 44.6, 31.3. MS (IE): m/z 244.1 [M+1H]⁺, 243.1, 148.1, 119.0. HRMS: Calculated for C₁₄H₁₇N₃O: 243.1372. Obtained: 243.1366.

### Obtaining 4-((2-(piperidin-1-yl)ethylamino)methyl)benzaldehyde (7{4}; X=H, m=2, Z= piperidin-1-yl):

Like 7{*1*} but using 2.01 g (9.4 mmol) of diethyl monoacetal of the terephthaldehyde (4a), 1.23 g (9.4 mmol) of 2-(piperidin-1-yl)ethylamine {*4*} and 0.36 g (9.4 mmol) of NaBH₄. 2.79 g (8.7 mmol, 93%) of a yellow oil corresponding to the acetal 8{*4*} are obtained. 2.75 g (8.6 mmol) of 8{*4*} are hydrolized with 2M HCl and 2.11 g (8.6 mmol, 100%) of a yellowish oil 7{*4*} are obtained. IR (film): ν (cm⁻¹) 3308, 3050, 2934, 2851, 2809, 1701, 1606, 779. ¹H-NMR (300 MHz, CDCl₃): δ (ppm) 10.00 (s, 1H, CHO), 7.84 (d, 2H, *J*=8.1 Hz, Ph), 7.50 (d, 2H, *J*=8.1 Hz, Ph), 3.89 (s, 2H, CH₂-Ph), 2.70 (t, 2H, *J*=6.2 Hz, CH₂-N), 2.47 (t, 2H, *J*=6.2 Hz, CH₂-N), 2.36 (bs, 4H, CH₂-N), 2.18 (bs, 1H, NH), 1.57 (m, 4H, *J*=5.7 Hz, CH₂), 1.43 (m, 2H, CH₂), ¹³C-NMR (75 MHz, CDCl₃): δ (ppm) 191.8, 147.7, 135.2, 129.8, 128.4, 58.4, 54.7, 53.7, 45.9, 25.9, 24.4. MS (IE): m/z 246.2 [M]⁺, 119.1, 98.1. HRMS: Calculated for C₁₅H₂₂N₂O: 246.1732. Obtained: 246.1731.

### Obtaining 4-((3-(2-methylpiperidin-1-yl)propylamino)methyl)benzaldehyde (7{5}; X=H, m=3, Z= 2-methylpiperidin-1-yl):

As for 7{*1*}, but using 2.01 g (9.3 mmol) of diethyl monoacetal of the terephthaldehyde (4a), 1.52 g (9.3 mmol) of 3-(2-methylpiperidin-1-yl)propan-l-amine {*5*} and 0.36 g (9.3 mmol) of NaBH₄. 3.18 g (9.1 mmol, 98%) of a yellow oil corresponding to the acetal 8{*5*} are obtained. 3.18 g (9.1 mmol) of 8{*5*} are hydrolized and 2.45 g (8.9 mmol, 98%) of a yellowish oil 7{*5*} are obtained. IR (film): ν (cm⁻¹) 3271, 2930, 2852, 2793, 2732, 1702, 1606, 1449, 1372, 781. ¹H-NMR (300 MHz, CDCl₃): δ (ppm) 10.00 (s, 1H, CHO), 7.85 (d, 2H, *J*=8.1 Hz, Ph), 7.50 (d, 2H, *J*=8.1 Hz, Ph), 3.87 (s, 2H, CH₂-Phᵣ), 2.87 (m, 1H, CH), 2.77 (m, 1H, CH₂), 2.65 (t, 2H, *J*=6.8 Hz, CH₂), 2.36 (m, 1H, CH₂), 2.27 (m, 1H, CH₂), 2.11 (m, 1H, CH₂), 2.00 (bs, 1 H, NH), 1.75-1.48 (m, 6H, CH₂), 1.29 (m, 2H, CH₂), 1.06 (d, 3H, *J*=6.0 Hz, CH₃). ¹³C-NMR (75 MHz, CDCl₃): δ (ppm) 191.8, 147.7, 135.2, 129.8, 128.4, 56.1, 53.8, 52.3, 52.0, 48.5, 34.6, 26.1, 25.9, 23.9, 19.0. MS (IE): m/z 275.2 [M+1H]⁺, 274.2, 119.0, 112.1. HRMS: Calculated for C₁₇H₂₆N₂O: 274.2045. Obtained: 274.2046.

### Obtaining 4-((3-(4-methylpiperazin-1-yl)propylamino)methyl)benzaldehyde (7{6}: X=H, m=3, Z= 4-methylpiperazin-1-yl):

As for 7{*1*}, but using 2.00 g (9.3 mmol) of diethyl monoacetal of the terephthaldehyde (4a), 1.49 g (9.3 mmol) of 3-(4-methylpiperazin-1-yl)propan-1-amine {6} and 0.36 g (9.3 mmol) of NaBH₄. 2.94 g (8.4 mmol, 90%) of a yellow oil corresponding to the acetal 8{*6*} are obtained. 2.93 g (8.4 mmol) of 8{*6*} are hydrolized with 2M HCl and 2.31 g (8.4 mmol, 100%) of a yellow oil 7{*6*} are obtained. IR (film): ν (cm⁻¹) 3276, 2936, 2875, 2794, 2769, 2740, 1700, 1606, 1458. ¹H-NMR (300 MHz, CDCl₃): δ (ppm) 10.00 (s, 1H, CHO), 7.85 (d, 2H, *J*=8.1 Hz, Ph), 7.50 (d, 2H, *J*=8.1 Hz, Ph), 3.87 (s, 2H, CH₂-Ph), 2.69 (t, 2H, *J*=6.9 Hz, CH₂-N), 2.45 (bs, 8H, CH₂-N), 2.42 (t, 2H, *J*=6.9 Hz, CH₂-N), 2.27 (s, 3H, CH₃), 2.08 (bs, 1H, NH), 1.72 (m, 2H, *J*=6.9 Hz, CH₂). ¹³C-NMR (75 MHz, CDCl₃): δ (ppm) 191.8, 147.7, 135.2, 129.8, 128.3, 57.0, 55.1, 53.7, 53.2, 48.3, 46.0, 27.0. MS (IE) : m/z 276.2 [M+1H]⁺, 275.2, 231.2. HRMS: Calculated for C₁₆H₂₅N₃O: 275.1998. Obtained: 275.2001.

### Obtaining 4-((2-morpholinoethylamino)methyl)benzaldehyde (7{7}; X=H, m=2, Z= morpholino):

As for 7{*1*}, but using 2.00 g (9.3 mmol) of diethyl monoacetal of the terephthaldehyde (4a), 1.23 g (9.3 mmol) of 2-morpholinoethylamine {*7*} and 0.36 g (9.3 mmol) of NaBH₄. 2.55 g (7.9 mmol, 85%) of a yellow oil corresponding to the acetal 8{*7*} are obtained. 2.52 g (7.8 mmol) of 8{*7*} are hydrolized with 2M HCl and 1.94 g (7.8 mmol, 100%) of a yellowish oil 7{*7*} are obtained. IR (film): ν (cm⁻¹) 3309, 2954, 2917, 2894, 2852, 2818, 1698, 1607, 1455, 1117, 824, 766. ¹H-NMR (300 MHz, CDCl₃): δ (ppm) 10.00 (s, 1H, CHO), 7.86 (d, 2H, *J*=8.1 Hz, Ph), 7.50 (d, 2H, *J*=8.1 Hz, Ph), 3.90 (s, 2H, CH₂-Ph), 3.70 (t, 4H, *J*=4.5 Hz, CH₂-O), 2.71 (t, 2H, *J*=6.0 Hz, CH₂-N), 2.51 (t, 2H, *J*=6.0 Hz, CH₂-N), 2.42 (t, 4H, *J*=4.5 Hz, CH₂-N), 1.93 (bs, 1 H, NH). ¹³C-NMR (75 MHz, CDCl₃): δ (ppm) 191.7, 147.5, 135.2, 129.8, 128.4, 66.9, 58.2, 53.7, 45.4. MS (IE): m/z 249.2 [M+1H]⁺, 248.2, 119.0, 100.0. HRMS: Calculated for C₁₄H₂₀N₂O₂: 248.1525. Obtained: 248.1531.

### Obtaining 4-((3-morpholinopropylamino)methyl)benzaldehyde (7{8}; X=H, m=3, Z= morpholino):

As for 7{*1*}, but using 2.01 g (9.3 mmol) of diethyl monoacetal of the terephthaldehyde (4a), 1.35 g (9.3 mmol) of 3-morpholinopropan-1-amine {*8*} and 0.36 g (9.3 mmol) of NaBH₄. 2.91 g (8.6 mmol, 92%) of a yellow oil corresponding to the acetal 8{*8*} are obtained. 2.86 g (8.5 mmol) of 8{*8*} are hydrolized and 1.60 g (6.1 mmol, 72%) of a yellow oil 7{*8*} are obtained. IR (film): ν (cm⁻¹) 3307, 2950, 2892, 2853, 2814, 1698, 1607, 1457, 1117, 861, 755. ¹H-NMR (300 MHz, CDCl₃): δ(ppm) 10.00 (s, 1H, CHO), 7.85 (d, 2H, *J*=8.1 Hz, Ph), 7.50 (d, 2H, *J*=8.1 Hz, Ph), 3.88 (s, 2H, CH₂-Ph), 3.70 (t, 4H, *J*=4.7 Hz, CH₂-O), 2.70 (t, 2H, *J*=6.9 Hz, CH₂-N), 2.45-2.39 (m, 6H, CH₂-N), 1.81 (bs, 1H, NH), 1.72 (m, 2H, *J*=6.9 Hz, CH₂). ¹³C-NMR (75 MHz, CDCl₃): δ (ppm) 191.2, 147.6, 135.2, 129.8, 128.3, 66.9, 57.3, 53.8, 53.7, 48.1, 26.7. MS (IE): m/z 262.2 [M]⁺, 84.0. HRMS: Calculated for C₁₅H₂₂N₂O₂: 262.1681. Obtained: 262.1682.

### Obtaining 4-((piperidin-1-ylimino)methyl)benzaldehyde (10{9}; X=H, m=0, Z= piperidin-1-yl):

1.00 g (7.4 mmol) of terephthaldehyde (9a) is dissolved in 30 mL of anhydrous MeOH and 4Å molecular sieve is added. A solution of 0.38 g (3.7 mmol) of 1-aminopiperidine {*9*} in 5 mL of anhydrous MeOH is added dropwise and under a nitrogen atmosphere. The mixture is stirred at reflux temperature for 36 h. The solvent is eliminated under reduced pressure and the obtained solid is purified by colunm chromatography (hexane/AcOEt 5:1). 0.48 g (2.2 mmol, 60%) of a yellow oil 10{*9*} are obtained. IR (CHCl₃ evaporated film): ν (cm⁻¹) 2938, 2854, 2818, 2731, 1694, 1605, 1579, 1549, 1448. ¹H-NMR (300 MHz, CDCl₃): δ (ppm) 9.96 (s, 1H, CHO), 7.83 (d, 2H, *J*=8.4 Hz, Ph), 7.71 (d, 2H, *J*=8.4 Hz, C_{Ar}H), 7.48 (s, 1H, CH=N), 3.25 (t, 4H, *J*=5.7 Hz, CH₂-N), 1.76 (m, 4H, *J*=5.7 Hz, CH₂), 1.61-1.54 (m, 2H, CH₂). ¹³C-NMR (75 MHz, CDCl₃): δ (ppm) 191.6, 142.8, 135.0, 131.0, 130.0, 125.8, 51.7, 25.1, 24.0. MS (IE): m/z 217.0 [M+1H]⁺, 216.0, 187.0, 132.0, 84.0. Anal. Calculated for C₁₃H₁₆N₂O_{:} C 72.19%, H 7.46%, N 12.95%, O 7.40%. Obtained: C 72.31%, H 7.56%, N12.88%.

### Obtaining 4-((2,6-dimethylpiperidin-1-ylimino)methyl)benzaldehyde (10{10}; X=H, m=0, Z= 2,6-dimethylpiperidin-1-yl):

As for 10{*9*}, but using 3.82 g (28.2 mmol) of terephthaldehyde (9a) and 2.01 g (14.1 mmol) of 1-amino-2,6-dimethylpiperidine {*10*}. It is purified by colunm chromatography (hexane/AcOEt 3:1) and 2.71 g (11.1 mmol, 78%) of a yellow oil 10{*10*} are obtained. IR (CHCl₃ evaporated film): ν (cm⁻¹) 2967, 2935, 2869, 2820, 2728, 1693, 1604, 1572, 1468, 1372. ¹H-NMR (300 MHz, CDCl₃): δ (ppm) 9.95 (s, 1 H, CHO), 7.81 (d, 2H, *J*=8.3 Hz, Ph), 7.69 (d, 2H, *J*=8.3 Hz, Ph), 7.35 (s, 1 H, CH=N), 3.92 (m, 2H, CH), 1.87-1.56 (m, 6H, CH₂), 1.15 (d, 6H, *J*=6.6 Hz, CH₃). ¹³C-NMR (75 MHz, CDCl₃): δ (ppm) 191.5, 143.5, 134.4, 130.0, 129.5, 125.3, 53.1, 30.8, 18.3, 15.6. MS (IE): m/z 245.2 [M+1H]⁺, 244.2, 89.0, 55.0. Anal. Calculated for C₁₅H₂₀N₂O: C 73.74%, H 8.25%, N 11.47%, O 6.55%. Obtained: C 73.38%, H 8.27%, N 11.41 %.

### Obtaining 4-((4-methylpiperazin-1-ylimino)methyl)benzaldehyde (10{11}; X=H, m=0, Z= 4-methylpiperazin-1-yl):

As for 10{*9*}, but using 1.14 (8.4 mmol) of terephthaldehyde (9a) and 0.50 g (4.2 mmol) of 1-amino-4-methylpiperazine {*11*}. It is purified by colunm chromatography (gradient of CH₂Cl₂ to CH₂Cl₂/MeOH 9:1). 0.80 g (3.5 mmol, 82%) of a yellow solid 10{*11*} are obtained. ¹H-NMR (300MHz, CDCl₃): δ (ppm) 9.98 (s, 1 H, CHO), 7.84 (d, 2H, *J*=7.7 Hz, Ph), 7.72 (d, 2H, *J*=7.7 Hz, Ph), 7.50 (s, 1 H, CH=N), 3.30 (t, 4H, *J*=5.1 Hz, CH₂-N), 2.62 (t, 4H, *J*=5.1 Hz, CH₂-N), 2.37 (s, 3H, CH₃).

### Obtaining N-(4-((2,6-dimethylpiperidin-1-ylimino)methyl)benzyl)-2-(pyrrolidin-1-yl)ethylamine (2{10,1}; X=H, m=0, n=2, Z=2,6-dimethylpiperidin-1-yl, Y=pyrrolidin-1-yl) :

0.52 g (2.1 mmol) of 10{*10*} and 0.25 g (2.1 mmol) of 2-(pyrrolidin-1-yl)ethylamine {*1*} are dissolved in 30 mL of anhydrous MeOH. 4Å molecular sieve is added and it is stirred at reflux temperature and under a nitrogen atmosphere for 36 h. The molecular sieve is filtered and 0.08 g (2.1 mmol) of NaBH₄ is added. It is allowed to react at room temperature for 16 h. Water is added and it is extracted with CH₂Cl₂. The organic phase is washed with brine and dried on MgSO₄. The solvent is eliminated under reduced pressure and 0.61 g (1.8 mmol, 85%) of a yellow oil **2**{*10*,*1*} are obtained.IR (film): ν (cm⁻¹) 3311, 2962, 2931, 2872, 2794, 1624,1459,1447,1369. ¹H-NMR (300 MHz, CDCl₃): δ (ppm) 8.07 (s, 1H, CH), 7.64 (d, 2H, *J*=8.1 Hz, Ph), 7.34 (d, 2H, *J*=8.1 Hz, Ph), 3.83 (s, 2H, CH₂-Ph), 3.06 (m, 2H, CH), 2.77 (t, 2H, *J*=6.0 Hz, CH₂-N), 2.63 (t, 2H, *J*=6.0 Hz, CH₂-N), 2.50 (m, 4H, CH₂-N), 2.34 (bs, 1H, NH), 1.77 (m, 8H, CH₂), 1.50 (m, 2H, CH₂), 1.00 (d, 6H, *J*=6.3 Hz, CH₃). MS (IE): m/z 342.3 [M]⁺, 84.0. HRMS: Calculated for C₂₁H₃₄N₄: 342.2783. Obtained: 342.2786.

### Obtaining N-(4-((2,6-dimethylpiperidin-1-ylimino)methyl)benzyl)-3-(1H-imidazol-1-yl)propan-1-amine (2{10,3}; X=H, m=0, n=3, Z=2,6-dimethylpiperidin-1-yl, Y=1H-imidazol-1-yl):

As for 2{*10*,*1*}, but using 0.49 g (2.0 mmol) of 10{*10*}, 0.26 g (2.0 mmol) of 3-(1*H*-imidazol-1-yl)propan-1-amine {*3*} and 0.08 g (2.0 mmol) of NaBH₄. 0.70 g (2.0 mmol, 100%) of a yellow oil 2{*10,3*} are obtained. IR (film): ν (cm⁻¹) 3284, 3106, 2961, 2931, 2867, 2856, 2824, 1624, 1508, 1449, 1369. ¹H-NMR (300 MHz, CDCl₃): δ (ppm) 8.04 (s, 1H, CH=N), 7.65 (d, 2H, *J*=8.1 Hz, Ph), 7.46 (s, 1H, CH), 7.31 (d, 2H, *J*=8.1 Hz, Ph), 7.04 (s, 1 H, CH), 6.89 (s, 1 H, CH), 4.05 (t, 2H, *J*=6.9 Hz, CH₂-N), 3.77 (s, 2H, CH₂-Ph), 3.10 (m, 2H, CH), 2.61 (t, 2H, *J*=6.9 Hz, CH₂-N), 1.93 (m, 2H, *J*=6.9 Hz, CH₂), 1.85 (bs, 1 H, NH), 1.77 (m, 4H, CH₂), 1.51 (m, 2H, CH₂), 1.00 (d, 6H, *J*=6.6 Hz, CH₃). ¹³C-NMR (75 MHz, CDCl₃): δ (ppm) 151.7, 141.4, 137.0, 134.0, 129.2, 128.2, 127.3, 118.7, 57.2, 53.7, 45.7, 44.7, 32.8, 31.4, 21.2, 20.5. MS (IE): m/z 354.2 [M+1H]⁺, 112.1, 81.0. HRMS: [M+1H]⁺ Calculated for C₂₁H₃₂N₅: 354.2658 [M+1H]⁺. Obtained: 354.2666.

### Obtaining N-(4-((2,6-dimethylpiperidin-1-ylimino)methyl)benzyl)-3-(4-methylpiperazin-1-yl)propan-1-amine (2{10,6}; X=H, m=0, n=3, Z=2,6-dimethylpiperidin-1-yl, Y=4-methylpiperazin-1-yl):

As for 2{*10*,*1*}, but using 0.47 g (1.9 mmol) of 10{*10*}, 0.31 g (1.9 mmol) of 3-(4-methylpiperazin-1-yl)propan-1-amine {*6*} and 0.07 g (1.9 mmol) of NaBH₄. 0.72 g (1.9 mmol, 97%) of a yellow oil 2{*10*,*6*} are obtained. IR (film): ν (cm⁻¹) 3286, 2932, 2872, 2837, 2794, 1625, 1458, 1370. ¹H-NMR (300 MHz, CDCl₃): δ (ppm) 8.05 (s, 1H, CH), 7.65 (d, 2H, *J*=8.1 Hz, Ph), 7.34 (d, 2H, *J*=8.1 Hz, Ph), 3.81 (s, 2H, CH₂-Ph), 3.08 (m, 2H, CH), 2.69 (t, 2H, *J*=6.9 Hz, CH₂-N), 2.44 (bs, 8H, CH₂-N), 2.41 (t, 2H, *J*=6.9 Hz, CH₂-N), 2.30 (bs, 1H, NH), 2.27 (s, 3H, CH₃), 1.81-1.68 (m, 6H, CH₂), 1.51 (m, 2H, CH₂), 1.00 (d, 6H, *J*=6.3 Hz, CH₃). ¹³C-NMR (75 MHz, CDCl₃): δ (ppm) 152.1, 141.3, 133.8, 128.2, 127.3, 57.2, 57.0, 55.1, 53.6, 53.2, 48.1, 46.0, 32.9, 26.7, 21.4, 20.6. MS (IE): m/z 386.3 [M+1H]⁺, 385.3, 273.2, 229.1, 113.0. HRMS: Calculated for C₂₃H₃₉N₅: 385.3205. Obtained: 385.3211.

### Obtaining N-(4-((2,6-dimethylpiperidin-1-ylimino)methyl)benzyl)-3-morpholinopropan-1-amine (2{10,8}; X=H, m=0, n=3, Z=2,6-dimethylpiperidin-1-yl, Y=morpholino):

As for 2{*10*,*1*}, but using 0.46 g (1.9 mmol) of 10{*10*}, 0.27 g (1.9 mmol) of 3-morpholinopropan-1-amine {*8*} and 0.07 g (1.9 mmol) of NaBH₄. 0.63 g (1.7 mmol, 91%) of a yellow oil 2{*10,8*} are obtained. IR (CHCl₃ evaporated film): ν (cm⁻¹) 3293, 2930, 2854, 2808, 1625, 1456, 1369, 1118, 863. ¹H-NMR (300 MHz, CDCl₃): δ (ppm) 8.06 (s, 1H, CH=N), 7.65 (d, 2H, *J*=8.1 Hz, Ph), 7.33 (d, 2H, *J*=8.1 Hz, Ph), 3.81 (s, 2H, CH₂-Ph), 3.70 (t, 4H, *J*=4.7 Hz, CH₂-O), 3.08 (m, 2H, CH), 2.69 (t, 2H, *J*=6.9 Hz, CH₂-N), 2.45-2.38 (m, 6H, CH₂-N), 2.06 (bs, 1 H, NH), 1.81-1.66 (m, 6H, CH₂), 1.51 (m, 2H, CH₂), 1.00 (d, 6H, *J*=6.3 Hz, CH₃). ¹³C-NMR (75 MHz, CDCl₃): δ (ppm) 152.2, 141.6, 133.8, 128.1, 127.3, 66.9, 57.4, 57.3, 53.8, 53.7, 47.9, 32.9, 26.6, 21.4, 20.6. MS (IE): m/z 372.2 [M]⁺, 143.1, 100.0. HRMS: Calculated for C₂₂H₃₆N₄O_{:} 372.2889. Obtained: 372.2895.

### Obtaining N-(4-((4-methylpiperazin-1-ylimino)methyl)benzyl)-2-(Pyrrolidin-1-yl)ethanamine (2{11,1}; X=H, m=0, n=3, Z=4-methylpiperazin-1-yl, Y= pyrrolidin-1-yl):

As for 2{*10*,*1*}, but using 0.32 g (1.4mmol) of 10{*11*}, 1.16 g of 2-(pyrrolidin-1-yl)ethylamine {*1*} and 0.053 g (1.4 mmol) of NaBH₄. 0.42 g (1.3 mmol, 92%) of a yellow oil 2{*11*,*1*} are obtained. IR (CHCl₃ evaporated film): ν (cm⁻¹) 3309, 2935, 2875, 2795, 1592, 1452. ¹H-NMR (300 MHz, CDCl₃): δ (ppm) 7.55 (d, 2H, *J*=8.0 Hz, Ph), 7.55 (s, 1 H, CH=N), 7.29 (d, 2H, *J*=8.0Hz, Ph), 3.80 (s, 2H, Ph-CH₂), 3.21 (t, 4H, *J*=5.0 Hz, CH₂-N), 2.73 (t, 2H, *J*=6.0 Hz, NH-CH₂), 2.61 (m, 6H, CH₂-N), 2.48 (m, 4H, CH₂-N), 2.36 (s, 3H, CH₃), 1.87 (s, 1 H, NH), 1.75 (m, 4H, CH₂). ¹³C-NMR (75 MHz, CDCl₃): δ (ppm) 140.4, 135.8, 134.8, 128.2, 126.0, 55.9, 54.5, 54.2, 53.8, 51.1, 47.8, 46.0, 23.5. MS (IE): m/z 330.4 [M+1H]⁺, 329.3, 245.2, 244.2, 216.2, 99.1, 84.0. Anal. Calculated for C₁₉H₃₁N₅: C 69.26%, H 9.48%, N 21.26%. Obtained: C 69.05%, H 9.61%, N 20,95%.

### Obtaining N-(4-((2-(Pyrrolidin-1-yl)ethylamino)methyl)benzyl)-3-(Pyrrolidin-1-yl)propan-1-amine (1{1,2}; X=H, m=2, n=3, Z=pyrrolidin-1-yl, Y= pyrrolidin-1-yl):

As for 2{*10*,*1*}, but using 0.81 g (3.5 mmol) of 9{*1*}, 0.46 g (3.5 mmol) of 3-(pyrrolidin-1-yl)propan-1-amine {*2*} and 0.13 g (3.5 mmol) of NaBH₄. 1.11 g (3.2 mmol, 92%) of a yellowish oil 1{*1*,*2*} are obtained. IR (film): ν (cm⁻¹) 3281, 2961, 2930, 2874, 2790, 1458, 1445. ¹H-NMR (300 MHz, CDCl₃): δ (ppm) 7.27 (s, 4H, PhH), 3.79 (s, 2H, CH₂-Ph), 3.77 (s, 2H, CH₂-Ph), 2.73 (m, 4H, CH₂-N), 2.61 (t, 2H, *J*=6.0 Hz, CH₂-N), 2.49 (m, 10H, CH₂-N), 2.14 (bs, 2H, NH), 1.79-1.71 (m, 10H, CH₂). ¹³C-NMR (75 MHz, CDCl₃): δ (ppm) 138.0, 138.8, 128.1, 128.0, 55.9, 54.7, 54.2, 53.8, 53.7, 48.0, 29.2, 23.5. MS (IE): m/z 345.3 [M+1H]⁺, 260.2, 84.1. HRMS: Calculated for C₂₁H₃₇N₄: 345.3018 [M+1H]⁺. Obtained: 345.3022.

### Obtaining N-(4-((2-(pyrrolidin-1-yl)ethylamino)methyl)benzyl)-3-(1H-imidazol-1-yl)propan-1-amine (1{3,1}; X=H, m=3, n=2, Z=pyrrolidin-1-yl, Y=1H imidazol-1-yl):

As for 2{*10*,*1*}, but using 0.89 g (3.7 mmol) of 9{*3*}, 0.43 g (3.7 mmol) of 2-(pyrrolidin-1-yl)ethylamine {*1*} and 0.14 g (3.7 mmol) of NaBH₄. 1.14 g (3.3 mmol, 91%) of a yellow oil 1{*3*,*1*} are obtained. IR (CHCl₃ evaporated film): ν (cm⁻¹) 3279, 3104, 2929, 2875, 2799, 1508, 1458, 1446. ¹H-NMR (300 MHz, CDCl₃): δ (ppm) 7.44 (s, 1H, CH), 7.27 (s, 4H, Ph), 7.03 (s, 1H, CH), 6.88 (s, 1H, CH), 4.04 (t, 2H, *J*=6.9 Hz, CH₂-N), 3.79 (s, 2H, CH₂-Ph), 3.73 (s, 2H, CH₂-Ph), 2.74 (t, 2H, *J*=5.9 Hz, CH₂-N), 2.61 (m, 4H, CH₂-N), 2.48 (m, 4H, CH₂-N), 2.18 (bs, 2H, NH), 1.92 (m, 2H, *J*=6.9 Hz,CH₂), 1.76 (m, 2H, *J*=3.3 Hz, CH₂). ¹³C-NMR (75 MHz, CDCl₃): δ (ppm) 139.1, 138.6, 137.0, 129.2, 128.2, 128.0, 118.7, 55.9, 54.2, 53.8, 53.7, 47.9, 45.6, 44.7, 31.4, 23.5. MS (IE): m/z 342.2 [M+1H]⁺, 257.2, 84.0. HRMS: Calculated for C₂₀H₃₂N_{5:} 342.2658 [M+1H]⁺. Obtained: 342.2666.

### Obtaining N-(4-((2-(pyrrolidin-1-yl)ethylamino)methyl)benzyl)-3-(4-methylpiperazin-1-yl)propan-1-amine (1{6,1}; X=H, m=3, n=2, Z=4-methylpiperazin-1-yl, Y= pyrrolidin-1-yl):

As for 2{*10*,*1*}, but using 0.91 g (3.3 mmol) of 9{*6*}, 0.39 g (3.3 mmol) of 2-(pyrrolidin-1-yl)ethylamine {*1*} and 0.13 g (3.3 mmol) of NaBH₄. 0.96 g (2.6 mmol, 77%) of a yellow oil 1{*6*,*1*} are obtained. IR (film): ν (cm⁻¹) 3288, 2934, 2875, 2793, 1458, 1447, 1372, 1354, 1015. ¹H-NMR (300 MHz, CDCl₃): δ (ppm) 7.27 (s, 4H, Ph), 3.79 (s, 2H, CH₂-Ph), 3.76 (s, 2H, CH₂-Ph), 2.74 (t, 2H, *J*=6.5 Hz, CH₂-N), 2.67 (t, 2H, *J*=6.9 Hz, CH₂-N), 2.61 (t, 2H, *J*=6.5 Hz, CH₂-N), 2.50-2.38 (m, 14H, CH₄-N), 2.27 (s, 3H, CH₃), 2.11 (bs, 2H, NH), 1.77-1.66 (m, 6H, CH₂). ¹³C-NMR (75 MHz, CDCl₃): δ (ppm) 138.9, 138.8, 128.1, 128.0, 57.0, 55.9, 55.1, 54.2, 53.8, 53.7, 53.2, 48.1, 47.9, 46.0, 27.0, 23.5. MS (IE): m/z 374.3 [M+1H]⁺, 303.2, 289.2, 113.1, 84.0. HRMS: Calculated for (C₂₂H₄₀N₅): 374.3284 [M+1H]⁺. Obtained: 374.3276.

### Obtaining N-(4-((2-(pyrrolidin-1-yl)ethylamino)methyl)benzyl)-3-morpholinopropan-1-amine (1{8,1}; X=H, m=3, n=2, Z=morpholino, Y= pyrrolidin-1-yl):

As for 2{*10*,*1*}, but using 0.84 g (3.2 mmol) of 9{*8*}, 0.37 g (3.2 mmol) of 2-(pyrrolidin-1-yl)ethylamine {*1*} and 0.12 g (3.2 mmol) of NaBH₄. 1.05 g (2.9 mmol, 91%) of a yellow oil 1{*8*,*1*} are obtained. IR (CHCl₃ evaporated film): ν (cm⁻¹) 3305, 2953, 2932, 2872, 2852, 2802, 1457, 1446, 1118, 862, 757. ¹H-NMR (300 MHz, CDCl₃): δ (ppm) 7.27 (s, 4H, Ph), 3.79 (s, 2H, CH₂-Ph), 3.77 (s, 2H, CH₂-Ph), 3.69 (t, 4H, 4.7 Hz, CH₂-O), 2.74 (t, 2H, *J*=5.9Hz, CH₂-N), 2.68 (t, 2H, *J*=6.9 Hz, CH₂-N), 2.61 (t, 2H, *J*=5.9 Hz, CH₂-N), 2.50-2.37 (m, 10H, CH₂-N), 2.04 (bs, 2H, NH), 1.80-1.68 (m, 6H, CH₂). ¹³C-NMR (75 MHz, CDCl₃): δ (ppm) 139.0, 138.7, 128.1, 128.0, 67.0, 57.4, 55.9, 54.2, 53.8, 53.7, 48.0, 47.9, 26.7, 23.5. MS (IE): m/z 361.2 [M+1H]⁺, 276.2, 100.0, 84.0. HRMS: Calculated for (C₂₁H₃₇N₄O): 361.2967 [M+1H]⁺. Obtained: 361.2965.

### Obtaining N-(4-((3-(1H-imidazol-1-yl)propylamino)methyl)benzyl)-3-(pyrrolidin-1-yl)propan-1-amine (1{3,2}; X=H, m=3, n=3, Z=1H-imidazol-1-yl, Y= pyrrolidin-1-yl):

As for 2{*10,1*}, but using 0.91 g (3.7 mmol) of 9{*3*}, 0.49 g (3.7 mmol) of 3-(pyrrolidin-1-yl)propan-1-amine {*2*} and 0.14 g (3.7 mmol) of NaBH₄. 1.33 g (3.7 mmol, 100%) of a yellowish oil 1{*3,2*} are obtained. IR (film): ν (cm⁻¹) 3281, 3104, 2931, 2875, 2794, 1508, 1458, 737. ¹H-NMR (300 MHz, CDCl₃): δ (ppm) 7.43 (s, 1H, CH), 7.27 (s, 4H, Ph), 7.03 (s, 1H, CH), 6.88 (s, 1H, CH), 4.04 (t, 2H, *J*=6.9 Hz, CH₂-N), 3.78 (s, 2H, CH₂-Ph), 3.74 (s, 2H, CH₂-Ph), 2.70 (t, 2H, *J*=6.9 Hz, CH₂-N), 2.60 (t, 2H, *J*=6.6 Hz, CH₂-N), 2.50 (m, 6H, CH₂-N), 2.05 (bs, 2H, NH), 1.92 (m, 2H, *J*=6.6 Hz, CH₂), 1.79-1.10 (m, 6H, CH₂). ¹³C-NMR (75 MHz, CDCl₃): δ (ppm) 139.1, 138.6, 137.0, 129.2, 128.1, 128.0, 118.7, 54.7, 54.2, 53.7, 48.0, 45.6, 44.7, 31.4, 29.2, 23.4. MS (IE): m/z 357.4 [M+ 2H]⁺, 356.4, 355.5, 84.2. HRMS: Calculated for C₂₁H₃₃N₅: 355.2736. Obtained: 355.2740.

### Obtaining N-(4-((2-(piperidin-1-yl)ethylamino)methyl)benzyl)-3-(pyrrolidin-1-yl)propan-1-amine (1{4,2}; X=H, m=2, n=3, Z= piperidin-1-yl, Y= pyrrolidin-1-yl):

As for 2{*10*,*1*}, but using 0.85 g (3.5 mmol) of 9{*4*}, 0.46 g (3.5 mmol) of 3-(pyrrolidin-1-yl)propan-1-amine {*2*} and 0.13 g (3.5 mmol) of NaBH₄. 1.13 g (3.2 mmol, 92%) of a yellowish oil 1{*4,2*} are obtained. IR (film): ν (cm⁻¹) 3298, 3047, 2933, 2876, 2851, 2792, 1454, 1443, 756. ¹H-NMR (300 MHz, CDCl₃): δ (ppm) 7.27 (s, 4H, Ph),3.78 (s, 2H, CH₂-Ph), 3.77 (s, 2H, CH₂-Ph), 2.69 (t, 4H, *J*=6.6 Hz, CH₂-N), 2.52 (m, 6H, CH₂-N), 2.44 (t, 2H, *J*=6.0 Hz, CH₂-N), 2.33 (bs, 4H, CH₂-N), 2.08 (bs, 2H, NH), 1.79-1.70 (m, 6H, CH₂), 1.55 (m, 4H, CH₂), 1.42 (m, 2H, CH₂), ¹³C-NMR (75 MHz, CDCl₃): δ (ppm) 138.9, 138.7, 128.0, 58.5, 54.7, 54.2, 53.7, 53.6, 48.0, 45.9, 29.2, 26.0, 24.5, 23.4. MS (IE): m/z 359.3 [M+1H]⁺, 260.2, 231.2, 98.2, 84.1. HRMS: Calculado C₂₂H₃₉N₄: 359.3175 [M+1H]⁺. Obtained: 359.3175.

### Obtaining N-(4-((3-(pyrrolidin-1-yl)propylamino)methyl)benzyl)-3-(2-methylpiperidin-1-yl)propan-1-amine (1{2,5}; X=H, m=3, n=3, Z= pyrrolidin-1-yl, Y=2-methylpiperidin-1:yl):

As for 2{*10*,*1*}, but using 0.96 g (3.9 mmol) of 9{*2*}, 0.63 g (3.9 mmol) of 3-(2-methylpiperidin-1-yl)propan-1-amine {*5*} and 0.15 g (3.9 mmol) of NaBH₄. 1.24 g (3.2 mmol, 83%) of a yellowish oil 1{*2,5*} are obtained. IR (film): ν (cm⁻¹) 3281, 3047, 2930, 2874, 2855, 2790, 1678, 1448, 1372, 755. ¹H-NMR (300 MHz, CDCl₃): δ (ppm) 7.27 (s, 4H, Ph), 3.77 (s, 2H, CH₂-Ph), 3.76 (s, 2H, CH₂-Ph), 2.86 (m, 1 H, CH), 2.68 (m, 5H, CH₂), 2.49 (m, 6H, CH₂), 2.36 (m, 1H, CH₂), 2.26 (m, 1H, CH₂), 2.11 (m, 1H, CH₂), 2.02 (bs, 2H, NH), 1.79-1.58 (m, 12H, CH₂), 1.27 (m, 2H, CH₂), 1.05 (d, 3H, *J*=6.3 Hz, CH₃). ¹³C-NMR (75 MHz, CDCl₃): δ (ppm) 138.9, 138.8, 128.1, 128.0, 56.0, 54.8, 54.3, 53.8, 53.7, 52.3, 52.1, 48.3, 48.0, 34.7, 29.3, 26.2, 25.8, 24.0, 23.5, 19.1. MS (IE): m/z 387.3 [M+1H]⁺, 386.3, 288.2, 259.2, 231.2, 112.1. HRMS: Calculated for C₂₄H₄₂N₄: 386.3409. Obtained: 386.3412.

### Obtaining N-(4-((3-(4-methylpiperazin-1-yl)propylamino)methyl)benzyl)-3-(pyrrolidin-1-yl)propan-1-amine (1{6,2}; X=H, m=3, n=3, Z= 4-methylpiperazin-1-yl, Y= pyrrolidin-1-yl):

As for 2{*10*,*1*}, but using 0.93 g (3.4 mmol) of 9{*6*}, 0.45 g (3.4 mmol) of 3-(pyrrolidin-1-yl)propan-1-amine {*2*} and 0.13 g (3.4 mmol) of NaBH₄. 1.25 g (3.2 mmol, 95%) of a yellowish oil 1{*6,2*} are obtained. IR (film): ν (cm⁻¹) 3284, 2935, 2875, 2792, 1458, 1372, 1353, 1014. ¹H-NMR (300 MHz, CDCl₃): δ (ppm) 7.27 (s, 4H, Ph), 3.77 (s, 2H, CH₂-Ph), 3.76 (s, 2H, CH₂-Ph), 2.68 (m, 4H, CH₂-N), 2.53-2.38 (m, 16H, CH₂-N), 2.27 (s, 3H, CH₃), 1.91 (bs, 2H, NH), 1.79-1.68 (m, 8H, CH₂). ¹³C-NMR (75 MHz, CDCl₃): δ (ppm) 138.9, 128.0, 56.9, 55.1, 54.7, 54.2, 53.7, 53.2, 48.1, 48.0, 29.3, 27.0, 23.5. MS (IE): m/z 387.4 [M]⁺, 289.3, 272.3, 260.2, 259.2, 231.3, 113.1, 84.1. HRMS: Calculated for C₂₃H₄₁N₅: 387.3362. Obtained: 387.3347.

### Obtaining N-(4-((2-morpholinoethylamino)methyl)benzyl)-3-(pyrrolidin-1-yl)propan-1-amine (1{2,7}; X=H, m=3, n=2, Z= pyrrolidin-1-yl, Y= morpholino):

As for 2{*10*,*1*}, but using 0.96 g (3.9 mmol) of 9{*2*}, 0.51 g (3.9 mmol) of 2-morpholinoethylamine {*7*} and 0.15 g (3.9 mmol) of NaBH₄. 1.27 g (3.5 mmol, 90%) of a yellowish oil 1{*2*,*7*} are obtained. IR (film): ν (cm⁻¹) 3304, 3046, 2935, 2872, 2852, 2800, 1675, 1454, 1118, 868, 766. ¹H-NMR (300 MHz, CDCl₃): δ (ppm) 7.28 (s, 4H, Ph), 3.79 (s, 4H, CH₂-Ph), 3.69 (t, 4H, 4.5 Hz, CH₂-O), 2.71 (m, 4H, CH₂-N), 2.51 (m, 8H, CH₂-N), 2.40 (t, 4H, *J*=4.5 Hz, CH₂-N), 2.18 (bs, 2H, NH), 1.78 (m, 6H, CH₂). ¹³C-NMR (75 MHz, CDCl₃): δ (ppm) 139.0, 138.6, 128.1, 67.0, 58.2, 54.8, 54.2, 53.7, 53.6, 48.0, 45.3, 28.9, 23.5. MS (IE): m/z 361.3 [M+1H]⁺, 360.3, 260.3, 231.2, 230.2, 100.0. HRMS: Calculated for C₂₁H₃₆N₄O: 360.2889. Obtained: 360.2879.

### Obtaining N-(4-((3-(pyrrolidin-1-yl)propylamino)methyl)benzyl)-3-morpholinopropan-1-amine (1{2,8}; X=H, m=3, n=3, Z= pyrrolidin-1-yl, Y= morpholino):

As for 2{*10,1*}, but using 0.94 g (3.8 mmol) of 9{*2*}, 0.55 g (3.8 mmol) of 3-morpholinopropan-1-amine {*8*} and 0.15 g (3.8 mmol) of NaBH₄. 1.33 g (3.5 mmol, 93%) of a yellow oil 1{*2*,*8*} are obtained. IR (film): ν (cm⁻¹) 3288, 3046, 2935, 2872, 2853, 2802, 1674, 1457, 1447, 1118, 862, 754. ¹H-NMR (300 MHz, CDCl₃): δ (ppm) 7.27 (s, 4H, Ph), 3.78 (s, 2H, CH₂-Ph), 3.77 (s, 2H, CH₂-Ph), 3.69 (t, 4H, *J*=4.8 Hz, CH₂-O), 2.70 (m, 4H, CH₂-N), 2.51 (m, 6H, CH₂-N), 2.41 (m, 6H, CH₂-N), 2.04 (bs, 2H, NH), 1.73 (m, 8H, CH₂). ¹³C-NMR (75 MHz, CDCl₃): δ (ppm) 138.9, 128.0, 67.0, 57.4, 54.8, 54.3, 53.8, 53.7, 48.1, 48.0, 29.3, 26.8, 23.5. MS (IE): m/z 374.3 [M+1H]⁺, 373.2, 231.2, 100.1. HRMS: Calculated for C₂₂H₃₈N₄O: 388.3202. Obtained: 388.3202.

### Obtaining N-(4-((2-(piperidin-1-yl)ethylamino)methyl)benzyl)-3-(1H-imidazol-1-yl)propan-1-amine (1{3,4}; X=H, m=3, n=2, Z= 1H-imidazol-1-yl, Y= piperidin-1-yl):

As for 2{*10*,*1*}, but using 0.90 g (3.7 mmol) of 9{*3*}, 0.48 g (3.7 mmol) of 2-(piperidin-1-yl)ethylamine {*4*} and 0.14 g (3.7 mmol) of NaBH₄. 1.23 g (3.5 mmol, 94%) of a yellow oil 1{*3,4*} are obtained. IR (molten film): ν (cm⁻¹) 3279, 2935, 2850, 2793, 1586, 1508, 1446. ¹H-NMR (300 MHz, CDCl₃): δ (ppm) 7.43 (s, 1H, CH), 7.28 (m, 4H, Ph), 7.03 (s, 1H, CH), 6.89 (s, 1H, CH), 4.04 (t, 2H, *J*=6.8 Hz, CH₂-N), 3.81 (s, 2H, CH₂-Ph), 3.74 (s, 2H, CH₂-Ph), 2.73 (t, 2H, *J*=5.9 Hz, CH₂-N), 2.60 (t, 2H, *J*=6.8 Hz, CH₂), 2.48 (t, 2H, *J*=5.9 Hz, CH₂-N), 2.46 (bs, 2H, NH), 2.37 (m, 6H, CH₂-N), 1.92 (m, 2H, *J*=6.8 Hz, CH₂), 1.56 (m, 4H, CH₂), 1.43 (m, 2H, CH₂). ¹³C-NMR (75 MHz, DMSO-d₆): δ (ppm) 137.0, 128.6, 128.3, 128.1, 119.1, 55.8, 53.7, 51.8, 51.3, 44.8, 43.9, 43.7, 29.9, 25.0, 23.6. MS (IE): m/z 356.1 [M+1H]⁺, 256.9, 98.0, 81.0. HRMS: Calculated for C₂₁H₃₄N_{5:} 356.2814. Obtained: 356.2809.

### Obtaining N-(4-((3-(1H-imidazol-1-yl)propylamino)methyl)benzyl)-3-(2-methylpiperidin-1-yl)propan-1-amine (1{3,5}; X=H, m=3, n=3, Z= 1H-imidazol-1-yl, Y= 2-methylpiperidin-1-yl):

As for 2{*10*,*1*}, but using 0.88 g (3.6 mmol) of 9{*3*}, 0.59 g (3.6 mmol) of 3-(2-methylpiperidin-1-yl)propan-1-amine {*5*} and 0.14 g (3.6 mmol) of NaBH₄. 1.20 g (3.1 mmol, 86%) of a yellow oil 1{*3,5*} are obtained. IR (CHCl₃ evaporated film): ν (cm⁻¹) 3277, 3104, 2929, 2853, 2802, 1508, 1450, 1373. ¹H-NMR (300 MHz, CDCl₃): δ (ppm) 7.44 (s, 1H, CH), 7.27 (s, 4H, Ph), 7.03 (s, 1H, CH), 6.89 (s, 1H, CH), 4.04 (t, 2H, *J*=6.9 Hz, CH₂-N), 3.77 (s, 2H, CH₂-Ph), 3.74 (s, 2H, CH₂-Ph), 2.87 (m, 1 H, CH), 2.74 (m, 1H, CH₂), 2.65 (t, 2H, *J*=6.9 Hz, CH₂), 2.60 (t, 2H, *J*=6.6 Hz, CH₂-N), 2.37 (m, 1H, CH₂), 2.27 (m, 1H, CH₂), 2.12 (m, 1H, CH₂), 2.09 (bs, 2H, NH), 1.92 (m, 2H, *J*=6.6 Hz, CH₂), 1.72-1.53 (m, 6H, CH₂), 1.29 (m, 2H, CH₂), 1.05 (d, 3H, *J*=6.3 Hz, CH₃). ¹³C-NMR (75 MHz, CDCl₃): δ (ppm) 138.9, 138.7, 137.1, 129.2, 128.2, 128.0, 118.7, 56.0, 53.7, 52.3, 52.0, 48.3, 45.7, 44.7, 34.6, 31.4, 26.1, 25.7, 23.9, 19.1. MS (IE): m/z 384.3 [M+1H]⁺, 383.3, 243.2, 155.2, 112.0.

### Obtaining N-(4-((3-(4-methylpiperazin-1-yl)propylamino)methyl)benzyl)-3-(1H-imidazol-1-yl)propan-1-amine (1{6,3}; X=H, m=3, n=3, Z=4-methylpiperazin-1-yl, Y=1H-imidazol-1-yl):

As for 2{*10*,*1*}, but using 0.76 g (2.8 mmol) of 9{*6*}, 0.35 g (2.8 mmol) of 3-(1*H*-imidazol-1-yl)propan-1-amine {*3*} and 0.11 g (2.8 mmol) of NaBH₄. 0.36 g (0.9 mmol, 34%) of a yellow oil 1{*6,3*} are obtained. IR (CHCl₃ evaporated film): ν (cm⁻¹) 3278, 2934, 2875, 2795, 1508, 1458, 1372, 1356, 1014. ¹H-NMR (300 MHz, CDCl₃): δ (ppm) 7.43 (s, 1H, CH), 7.27 (s, 4H, Ph), 7.03 (s, 1H, CH), 6.88 (s, 1H, CH), 4.04 (t, 2H, *J*=6.9 Hz, CH₂-N), 3.77 (s, 2H, CH₂-Ph), 3.74 (s, 2H, CH₂-Ph), 2.68 (t, 2H, *J*=7.2 Hz, CH₂-N), 2.61 (t, 2H, *J*=6.6 Hz, CH₂-N), 2.43 (bs, 8H, CH₂-N), 2.41 (t, 2H, *J*=7.2 Hz, CH₂-N), 2.27 (s, 3H, CH₃), 1.93 (bs, 2H, NH), 1.92 (m, 2H, *J*=6.6 Hz, CH₂), 1.71 (m, 2H, *J*=7.2 Hz, CH₂). ¹³C-NMR (75 MHz, CDCl₃): δ (ppm) 139.0, 138.7, 137.1, 129.2, 128.1, 128.0, 118.7, 56.9, 55.1, 53.7, 53.2, 48.1, 46.0, 45.7, 44.7, 31.4, 26.9. MS (IE): m/z 385.3 [M+1H]⁺, 271.2, 257.1, 113.1, 70.0. HRMS: Calculated for C₂₂H₃₆N₆: 384.3001. Obtained: 384.3004.

### Obtaining N-(4-((2-morpholinoethylamino)methyl)benzyl)-3-(1H-imidazol-1-yl)propan-1-amine (1{3,7}; X=H, m=3, n=3, Z=1H-imidazol-1-yl, Y=morpholino):

As for 2{*10*,*1*}, but using 0.79 g (3.2 mmol) of 9{*3*}, 0.43 g (3.2 mmol) of 2-morpholinoethylamine {*7*} and 0.12 g (3.2 mmol) of NaBH₄. 1.11 g (3.1 mmol, 96%) of a yellowish oil 1{*3*,*7*} are obtained. IR (film): ν (cm⁻¹) 3299, 3105, 2934, 2890, 2851, 2811, 1508, 1454, 1117, 854. ¹H-NMR (300 MHz, CDCl₃): δ (ppm) 7.43 (s, 1H, CH), 7.28 (s, 4H, Ph), 7.03 (s, 1H, CH), 6.88 (s, 1H, CH), 4.05 (t, 2H, *J*=6.9 Hz, CH₂-N), 3.79 (s, 2H, CH₂-Ph), 3.74 (s, 2H, CH₂-Ph), 3.69 (t, 4H, *J*=4.7 Hz, CH₂-O), 2.71 (t, 2H, *J*=6.0 Hz, CH₂-N), 2.60 (t, 2H, *J*=6.9 Hz, CH₂-N), 2.50 (t, 2H, *J*=6.0 Hz, CH₂-N), 2.41 (t, 4H, *J*=4.7 Hz, CH₂-N), 1.95 (bs, 2H, NH), 1.93 (m, 2H, *J*=6.9 Hz, CH₂). ¹³C-NMR (75 MHz, CDCl₃): δ (ppm) 139.0, 138.7, 137.0, 129.2, 128.1, 128.0, 118.7, 67.0, 58.2, 53.7, 45.6, 45.3, 44.6, 31.2. MS (IE): m/z 358.2 [M+1H]⁺, 257.0, 100.0. HRMS: Calculated for C₂₀H₃₁N₅O: 357.2529. Obtained: 357.2517.

### Obtaining N-(4-((3-morpholinopropylamino)methyl)benzyl)-3-(1H-imidazol-1-yl)propan-1-amine (1{8,3}; X=H, m=3, n=3, Z=morpholino, Y=1H-imidazol-1-yl):

As for 2{*10*,*1*}, but using 1.15 g (4.4 mmol) of 9{*8*}, 0.56 g (4.4 mmol) of 3-(1*H*-imidazol-1-yl)propan-1-amine {*3*} and 0.17 g (4.4 mmol) of NaBH₄. 1.36 g (3.7 mmol, 85%) of a yellow oil 1{*8*,*3*} are obtained. IR (CHCl₃ evaporated film): ν (cm⁻¹) 3282, 2935, 2852, 2808, 1509, 1456, 1117. ¹H-NMR (300 MHz, CDCl₃): δ (ppm) 7.42 (s, 1H, CH), 7.27 (m, 4H, Ph), 7.03 (s, 1H, CH), 6.88 (s, 1H, CH), 4.04 (t, 2H, *J*=6.9 Hz, CH₂-N), 3.79 (s, 2H, CH₂-Ph), 3.74 (s, 2H, CH₂-Ph), 3.69 (t, 4H, *J*=4.7 Hz, CH₂-O), 2.71 (t, 2H, *J*=6.9 Hz, CH₂), 2.60 (t, 2H, *J*=6.9 Hz, CH₂), 2.42 (m, 6H, CH₂-N), 1.92 (m, 2H, *J*=6.9 Hz, CH₂), 1.88 (s, 2H, NH), 1.72 (m, 2H, *J=*6.9 Hz, CH₂), ¹³C-NMR (75 MHz, CDCl₃): δ (ppm) 138.9, 138.18, 137.0, 129.1, 128.2, 128.2, 118.7, 66.9, 57.4, 53.7, 53.6, 53.4, 48.0, 45.5, 44.6, 31.3, 26.1. MS (IE): m/z 373.0 [M+2H]⁺, 372.0, 114.0, 100.0, 95.0, 81.0. HRMS: Calculated for C₂₁H₃₄N₅O: 372.2763 [M+1H]⁺. Obtained: 372.2758.

### Obtaining N-(4-((2-(piperidin-1-yl)ethylamino)methyl)benzyl)-3-(4-methylpiperazin-1-yl)propan-1-amine (1{6,4}; X=H, m=3, n=2, Z=4-methylpiperazin-1-yl, Y=piperidin-1-yl):

As for 2{*10*,*1*}, but using 0.73 g (2.6 mmol) of 9{*6*}, 0.34 g (2.6 mmol) of 2-(piperidin-1-yl)ethylamine {*4*} and 0.10 g (2.6 mmol) of NaBH₄. 0.48 g (1.2 mmol, 47%) of a yellowish oil 1{*6,4*} are obtained. IR (CHCl₃ evaporated film): ν (cm⁻¹) 3285, 2934, 2878, 2849, 2794, 1457, 1446, 1372, 1349, 1015. ¹H-NMR (300 MHz, CDCl₃): δ (ppm) 7.27 (s, 4H, Ph), 3.78 (s, 2H, CH₂-Ph), 3.77 (s, 2H, CH₂-Ph), 2.69 (m, 4H, CH₂-N), 2.47-2.34 (m, 16H, CH₂-N), 2.27 (s, 3H, CH₃), 2.22 (bs, 2H, NH), 1.72 (m, 2H, *J*=6.9 Hz, CH₂), 1.55 (m, 4H, CH₂), 1.42 (m, 2H, CH₂). ¹³C-NMR (75 MHz, CDCl₃): δ (ppm) 139.0, 138.5, 128.1, 128.8, 58.5, 57.0, 55.1, 54.7, 53.7, 53.6, 53.2, 48.1, 46.0, 45.9, 26.8, 26.0, 24.5.

### Obtaining N-(4-((2-(piperidin-1-yl)ethylamino)methyl)benzyl)-3-morpholinopropan-1-amine (1{8,4}; X=H, m=3, n=2, Z=morpholino, Y=piperidin-1-yl):

As for 2{*10*,*1*}, but using 0.89 g (3.4 mmol) of 9{*8*}, 0.44 g (3.4 mmol) of 2-(piperidin-1-yl)ethylamine {*4*} and 0.13 g (3.4 mmol) of NaBH₄. 1.24 g (3.3 mmol, 98%) of a yellow oil 1{*8*,*4*} are obtained. IR (CHCl₃ evaporated film): ν (cm⁻¹) 3304, 2933, 2852, 2806, 1454, 1444, 1119, 862, 756. ¹H-NMR (300 MHz, CDCl₃): δ (ppm) 7.27 (s, 4H, Ph), 3.78 (s, 2H, CH₂-Ph), 3.77 (s, 2H, CH₂-Ph), 3.69 (t, 4H, *J*=4.7 Hz, CH₂-O), 2.69 (m, 4H, CH₂-N), 2.47-2-34 (m, 12H, CH₂-N), 2.11 (bs, 2H, NH), 1.71 (m, 2H, *J*=7.2 Hz, CH₂), 1.55 (m, 4H, CH₂), 1.42 (m, 2H, CH₂). ¹³C-NMR (75 MHz, CDCl₃): δ (ppm) 139.0, 138.6, 128.1, 128.0, 66.9, 58.5, 57.4, 54.7, 53.8, 53.7, 48.0, 45.9, 26.6, 26.0, 24.5. MS (IE): m/z 373.3 [M-1H]⁺, 98.0. Anal. Calculated for C₂₂H₃₈N₄O: C 70.54%, H 10.23%, N 14.96%, O 4.27%. Obtained: C 70.68%, H 10.05%, N 14.67%.

### Obtaining N-(4-((3-(4-methylpiperazin-1-yl)propylamino)methyl)benzyl)-3-(2-methylpiperidin-1-yl)propan-1-amine (1{6,5}; X=H, m=3, n=3, Z=4-methylpiperazin-1-yl, Y=2-methylpiperidin-1-yl):

As for 2{*10,1*}, but using 0.75 g (2.7 mmol) of 9{*6*}, 0.45 g (2.7 mmol) of 3-(2-methylpiperidin-1-yl)propan-1-amine {*5*} and 0.10 g (2.7 mmol) of NaBH₄. 0.45 g (1.1 mmol, 39%) of a yellow oil 1{*6,5*} are obtained. IR (CHCl₃ evaporated film): v (cm⁻¹) 3280, 2931, 2875, 2852, 2793, 1458, 1448, 1372, 1015. ¹H-NMR (300 MHz, CDCl₃): δ (ppm) 7.28 (s, 4H, Ph), 3.77 (s, 4H, CH₂-Ph), 2.88 (m, 1H, CH), 2.75 (m, 1 H, CH₂), 2.67 (t, 2H, *J*=6.6 Hz, CH₂-N), 2.65 (t, 2H, *J*=6.6 Hz, CH₂-N), 2.43 (bs, 12H, CH₂-N), 2.27 (s, 3H, CH₃), 2.23 (bs, 2H, NH), 2.12 (m, 1H, CH₂), 1.76-1.53 (m, 8H, CH₂), 1.32-1.21 (m, 2H, CH₂), 1.05 (d, 3H, *J*=6.3 Hz, CH₃). ¹³C-NMR (75 MHz, CDCl₃): δ (ppm) 138.8, 138.6, 128.1, 128.0, 57.0, 56-0, 55.1, 53.7, 53.2, 52.3, 52.0, 48.3, 48.1, 46.0, 34.5, 26.9, 26.0, 25.6, 23.9, 19.0. MS (IE): m/z 415.4 [M]⁺, 218.2, 112.2, 98.0. HRMS: Calculated for C₂₅H₄₅N₅: 415.3675. Obtained: 415.3660.

### Obtaining N-(4-((2-morpholinoethylamino)methyl)benzyl)-3-(2-methylpiperidin-1-yl)propan-1-amine (1{5,7}; X=H, m=3, n=2, Z=2-methylpiperidin-1-yl, Y=morpholino):

As for 2{*10,1*}, but using 1.35 g (8.5 mmol) of 9{*5*}, 1.12 g (8.5 mmol) of 2-morpholinoethylamine {*7*} and 0.33 g (8.5 mmol) of NaBH₄. 2.46 g (6.3 mmol, 74%) of a yellowish oil 1{*5,7*} are obtained. IR (CHCl₃ evaporated film): ν (cm⁻¹) 3305, 2930, 2853, 2807, 1453, 1119. ¹H-NMR (300 MHz, CDCl₃): δ (ppm) 7.27 (s, 4H, Ph), 3.79 (s, 2H, CH₂-Ph), 3.77 (s, 2H, CH₂-Ph), 3.69 (t, 4H, *J*=4.5 Hz, CH₂-O), 2.86 (m, 1H, CH), 2.74 (m, 1H, CH₂), 2.70 (t, 2H, *J*=6.0 Hz, CH₂-N), 2.633 (t, 2H, *J*=6.9 Hz, CH₂-N), 2.49 (t, 2H, *J*=6.0 Hz, CH₂-N), 2.40 (m, 4H, CH₂-N), 2.34 (m, 1H, CH₂), 2.25 (m, 1H, CH₂), 2.11 (m, 1H, CH₂), 1.91 (bs, 2H, NH), 1.63 (m, 6H, CH₂), 1.27 (m, 2H, CH₂), 1.05 (d, 3H, *J*=6.0 Hz, CH₃). ¹³C-NMR (75 MHz, CDCl₃): δ (ppm) 139.0, 138.9, 128.0, 128.0, 67.0, 58.3, 55.9, 53.8, 53.7, 53.7, 52.3, 52.1, 48.4, 45.3, 34.7, 26.2, 25.8, 24.0, 19.2. MS (IE): m/z 389.3 [M+1H]⁺, 155.2, 126.2, 112.1, 100.0, 98.0. HRMS: Calculated for C₂₃H₄₀N₄O: 374.3046. Obtained: 374.3049.

### Obtaining N-(4-((3-morpholinopropylamino)methyl)benzyl)-3-(2-methylpiperidin-1-yl)propan-1-amine (1{8,5}; X=H, m=3, n=3, Z=morpholino, Y=2-methylpiperidin-1-yl):

As for 2{*10*,*1*}, but using 1.00 g (3.8 mmol) of 9{*8*}, 0.62 g (3.8 mmol) of 3-(2-methylpiperidin-1-yl)propan-1-amine {*5*} and 0.15 g (3.8 mmol) of NaBH₄. 1.54 g (3.8 mmol, 100%) of a yellow oil 1{*8,5*} are obtained. IR (CHCl₃ evaporated film): ν (cm⁻¹) 3286, 2929, 2853, 2806, 1448, 1372, 1119, 862, 752. ¹H-NMR (300 MHz, CDCl₃): δ (ppm) 7.27 (s, 4H, Ph), 3.77 (s, 4H, CH₂-Ph), 3.70 (t, 4H, *J*=4.7 Hz, CH₂-O), 2.87 (m, 1H, CH), 2.79-2.62 (m, 5H, CH₂), 2.43-2.32 (m, 7H, CH₂), 2.27(m, 1H, CH₂), 2.12 (bs, 2H, NH), 2.11 (m, 1H, CH₂), 1.75-1.52 (m, 8H, CH₂), 1.30 (m, 2H, CH₂), 1.05 (d, 3H, *J*=6.0 Hz, CH₃). ¹³C-NMR (75 MHz, CDCl₃): δ (ppm) 138.9, 138.7, 128.1, 128.0, 67.0, 57.4, 56.0, 53.8, 53.7, 52.3, 52.0, 48.3, 48.0, 34.6, 27.0, 26.1, 25.7, 23.9, 19.1. MS (IE): m/z 403.4 [M+1H]⁺, 112.0, 100.0. HRMS: Calculated for C₂₄H₄₂N₄O: 402.3359. Obtained: 402.3354.

### Obtaining N-(4-((2-morpholinoethylamino)methyl)benzyl)-3-(4-methylpiperazin-1-yl)propan-1-amine (1{7,6}; X=H, m=2, n=3, Z=morpholino, Y=4-methylpiperazin-1-yl):

As for 2{*10,1*}, but using 0.68 g (2.7 mmol) of 9{*7*}, 0.44 g (2.7 mmol) of 3-(4-methylpiperazin-1-yl)propan-1-amine {*6*} and 0.10 g (2.7 mmol) of NaBH₄. 0.92 g (2.4 mmol, 86%) of a yellow oil 1{*7,6*} are obtained. IR (film): ν (cm⁻¹) 3300, 2935, 2872, 2796, 1456, 1372, 1355, 1118, 1014, 868, 765. ¹H-NMR (300 MHz, CDCl₃): δ (ppm) 7.27 (s, 4H, Ph), 3.79 (s, 2H, CH₂-Ph), 3.77 (s, 2H, CH₂-Ph), 3.68 (t, 4H, *J*=4.7 Hz, CH₂-O), 2.69 (m, 4H, CH₂-N), 2.52-2.39 (m, 16H, CH₂-N), 2.27 (s, 3H, CH₃), 2.11 (bs, 2H, NH), 1.72 (m, 2H, *J*=6.9 Hz, CH₂). ¹³C-NMR (75 MHz, CDCl₃): δ (ppm) 138.9, 138.7, 128.0, 67.0, 58.2, 57.0, 55.1, 53.7, 53.2, 48.1, 46.0, 45.3, 26.9. MS (IE): m/z 389.4 [M]⁺, 100.1. HRMS: Calculated for C₂₂H₃₉N₅O: 389.3155. Obtained: 389.3153.

### Obtaining N-(4-((3-morpholinopropylamino)methyl)benzyl)-3-(4-methylpiperazin-1-yl)propan-1-amine (1{8,6}; X=H, m=3, n=3, Z=morpholino, Y=4-methylpiperazin-1-yl):

As for 2{*10,*1}, but using 0.71 g (2.7 mmol) of 9{*8*}, 0.43 g (2.7 mmol) of 3-(4-methylpiperazin-1-yl)propan-1-amine {*6*} and 0.10 g (2.7 mmol) of NaBH₄. 0.91 g (2.3 mmol, 84%) of a yellow oil 1{*8,6*} are obtained. IR (film): ν (cm⁻¹) 3288, 2935, 2872, 2851, 2795, 1476, 1372, 1356, 1118, 1014, 862, 751. ¹H-NMR (300 MHz, CDCl₃): δ (ppm) 7.27 (s, 4H, Ph), 3.77 (s, 4H, CH₂-Ph), 3.69 (t, 4H, *J*=4.8 Hz, CH₂-O), 2.68 (t, 2H, *J*=6.9 Hz, CH₂-N), 2.67 (t, 2H, *J*=6.9 Hz, CH₂-N), 2.45-2.37 (m, 16H, CH₂-N), 2.27 (s, 3H, CH₃), 2.03 (bs, 2H, NH), 1.71 (m, 4H, *J*=6.9 Hz, CH₂). ¹³C-NMR (75 MHz, CDCl₃): δ (ppm) 138.8, 128.0, 127.9, 66.9, 57.3, 56.9, 55.1, 53.7, 53.2, 48.1, 47.9, 46.0, 26.9, 26.7. MS (IE): m/z 403.4 [M]⁺, 113.2, 100.1. HRMS: Calculated for C₂₃H₄₁N₅O: 403.3311. Obtained: 403.3311.

### Obtaining N-(4-((2-morpholinoethylamino)methyl)benzyl)-3-morpholinopropan-1-amine (1{8,7}: X=H, m=3, n=2, Z=morpholino, Y=morpholino):

As for 2{*10,1*}, but using 0.88 g (3.3 mmol) of 9{*8*}, 0.44 g (3.3 mmol) of 2-morpholinoethylamine {*7*} and 0.13 g (3.3 mmol) of NaBH₄. 1.16 g (3.1 mmol, 92%) of an orange oil 1{*8,7*} are obtained. IR (CHCl₃ evaporated film): ν (cm⁻¹) 3307, 2950, 2891, 2852, 2808, 1455, 1118, 863, 765. ¹H-NMR (300 MHz, CDCl₃): δ (ppm) 7.27 (s, 4H, Ph), 3.79 (s, 2H, CH₂-Ph), 3.77 (s, 2H, CH₂-Ph), 3.69 (m, 8H, CH₂-O), 2.69 (m, 4H, CH₂-N), 2.50 (t, 2H, *J*=6.0 Hz, CH₂-N), 2.45-2.38 (m, 10H, CH₂-N), 1.96 (bs, 2H, NH), 1.71 (m, 2H, *J*=6.9 Hz, CH₂). ¹³C-NMR (75 MHz, CDCl₃): δ (ppm) 138.9, 138.8, 128.1, 128.0, 67.0, 58.2, 57.4, 53.8, 53.7, 48.0, 45.3, 26.7. MS (IE): m/z 377.2 [M+1H]⁺, 376.2, 276.2, 100.0. HRMS: Calculated for C₂₁H₃₆N₄O₂: 376.2838. Obtained: 376.2849.

### Obtaining ((4-(N-(4-methylpiperazin-1-yl)imino)methyl)phenyl)-N-(piperidin-1-yl)methanimine (3{11,9}; X=H, m=n=0, Z=4-methylpiperazin-1-yl, Y= piperidin-1-yl):

0.53 g (2.3 mmol) of 10{*11*} and 0.24 g (2.3 mmol) of 1-aminopiperidine {*9*} are dissolved in 30 mL of anhydrous MeOH. 4Å molecular sieve is added and it is stirred at reflux temperature and under a nitrogen atmosphere for 36 h. The molecular sieve is filtered and the solvent is eliminated under reduced pressure and 0.69 g (2.2 mmol, 95%) of a yellow solid 3{*11*,*9*} are obtained. IR (film): ν (cm⁻¹) 2934, 2837, 2798, 1577, 1452, 1365, 1001. ¹H-NMR (300 MHz, CDCl₃): δ (ppm) 7.56 (s, 4H, Ph), 7.53 (s, 1 H, CH=N), 7.52 (s, 1 H, CH=N), 3.22 (t, 4H, *J*=5.1 Hz, CH₂-N), 3.17 (t, 4H, *J*=5.6 Hz, CH₂-N), 2.62 (t, 4H, *J*=5.1 Hz, CH₂-N), 2.36 (s, 3H, CH₃), 1.75 (m, 4H, CH₂), 1.54 (m, 2H, CH₂). ¹³C-NMR (75 MHz, CDCl₃): δ (ppm) 136.4, 135.6, 135.5, 134.0, 126.2, 126.0, 54.6, 52.1, 51.0, 46.0, 25.3, 24.2. MS (IE): m/z 314.2 [M+1H]⁺, 313.2, 99.2. HRMS: Calculated for C₁₈H₂₇N₅: 313.2266. Obtained: 313.2266.

### Obtaining N-(4-((piperidin-1-ylimino)methyl)benzyl)-3-(pyrrolidin-1-yl)propan-1-amine (2{2,9}; X=H, m=3, n=0, Z= pyrrolidin-1-yl, Y= piperidin-1-yl):

As for 3{*11,9*}, but using 1.04 g (4.2 mmol) of 9{*2*} and 0.44 g (4.2 mmol) 1-aminopiperidine {*9*}. 1.17 g (3.6 mmol, 84%) of a yellow oil 2{*2,9*} are obtained. IR (film): ν (cm⁻¹) 3287, 2935, 2874, 2854, 2793, 1591, 1450. ¹H-NMR (300 MHz, CDCl₃): δ (ppm) 7.55 (d, 2H, *J*=8.1 Hz, Ph), 7.54 (s, 1H, CH=N), 7.27 (d, 2H, *J*=8.1 Hz, Ph), 3.78 (s, 2H, CH₂-Ph), 3.15 (t, 4H, *J*=5.7 Hz, CH₂-N), 2.28 (t, 2H, *J*=6.9 Hz, CH₂-N), 2.50 (m, 6H, CH₂-N), 2.03 (bs, 1H, NH), 1.79-1.69 (m, 10H, CH₂), 1.55 (m, 2H, CH₂). ¹³C-NMR (75 MHz, CDCl₃): δ (ppm) 139.7, 135.3, 134.5, 128.1, 125.9, 54.8, 54.2, 53.7, 52.1, 48.0, 29.1, 25.2, 24.2, 23.5. MS (IE): m/z 328.3 [M]⁺, 244.2, 201.2, 84.1. HRMS: Calculated for C₂₀H₃₂N₄: 328.2627. Obtained: 328.2624.

### Obtaining N-(4-((piperidin-1-ylimino)methyl)benzyl)-3-(1H-imidazol-1-yl)propan-1-amine (2{3,9}; X=H, m=3, n=0, Z=1H-imidazol-1-yl, Y=piperidin-1-yl):

As for 3{*11,9*}, but using 0.98 g (4.0 mmol) of 9{*3*} and 0.42 g (4.0 mmol) of 1-aminopiperidine {*9*}. 1.31 g (4.0 mmol, 100%) of a reddish oil 2{*3,9*} are obtained. IR (CHCl₃ evaporated film): ν (cm⁻¹) 3282, 3105, 2935, 2853, 2809, 1590, 1508, 1450. ¹H-NMR (300 MHz, CDCl₃): δ (ppm) 7.56 (s, 1H, CH=N), 7.55 (d, 2H, *J*=8.1 Hz, Ph), 7.45 (s, 1H, CH), 7.26 (d, 2H, *J*=8.1 Hz, Ph), 7.03 (s, 1H, CH), 6.88 (s, 1H, CH), 4.03 (t, 2H, *J*=6.9 Hz, CH₂-N), 3.74 (s, 2H, CH₂-Ph), 3.15 (t, 4H, *J*=5.6 Hz, CH₂-N), 2.60 (t, 2H, *J*=6.9 Hz, CH₂-N), 2.07 (bs, 1 H, NH), 1.92 (m, 2H, *J*=6.9 Hz, CH₂), 1.75 (m, 4H, *J*=5.7 Hz, CH₂), 1.54 (m, 2H, *J*=5.7 Hz, CH₂). ¹³C-NMR (75 MHz, CDCl₃): δ (ppm) 139.5, 137.0, 135.5, 134.2, 129.2, 128.1, 125.9, 118.7, 53.7, 52.1, 45.6, 44.7, 31.3, 25.2, 24.2. MS (IE): m/z 326.2 [M+1H]⁺, 243.2, 215.1, 91.0. HRMS: Calculated for C₁₉H₂₇N₅: 325.2266. Obtained: 325.2277.

### Obtaining N-(4-((piperidin-1-ylimino)methyl)benzyl)-3-(2-methylpiperidin-1-yl)propan-1-amine (2{5,9}; X=H, m=3, n=0, Z= 2-methylpiperidin-1-yl, Y= piperidin-1-yl):

As for 3{*11,9*}, but using 0.51 g (1.9 mmol) of 9{*5*} and 0.19 g (1.9 mmol) of 1-aminopiperidine {*9*}. 0.67 g (1.9 mmol, 100%) of a reddish oil 2{*5,9*} are obtained. IR (film): ν (cm⁻¹) 3282, 2932, 2854, 2804, 1591, 1450, 1364, 992. ¹H-NMR (300 MHz, CDCl₃): δ (ppm) 7.55 (d, 2H, *J*=8.1 Hz, Ph), 7.54 (s, 1H, CH=N), 7.28 (d, 2H, *J*=8.1 Hz, Ph), 3.78 (s, 2H, CH₂-Ph), 3.15 (t, 4H, *J*=5.4 Hz, CH₂-N), 2.87 (m, 1 H, CH), 2.74 (m, 1H, CH₂), 2.65 (t, 2H, *J*=6.9 Hz, CH₂), 2.42 (bs, 1H, NH), 2.37 (m, 1H, CH₂), 2.28 (m, 1H, CH₂), 2.13 (m, 1H, CH₂), 1.79-1.52 (m, 12H, CH₂), 1.29 (m, 2H, CH₂), 1.05 (d, 3H, *J*=6.3 Hz, CH₃)- ¹³C-NMR (75 MHz, CDCl₃): δ (ppm) 139.5, 135.4, 134.4, 128.2, 125.9, 56.0, 53.7, 52.3, 52.1, 52.0, 48.3, 34.6, 26.1, 25.5, 25.3, 24.2, 23.9, 19.0. MS (IE): m/z 356.2 [M]⁺, 216.0, 201.0, 155.1, 112.0, 84.0. HRMS: Calculated for C₂₂H₃₆N_{4:} 356.2940. Obtained: 356.2942.

### Obtaining N-(4-((piperidin-1-ylimino)methyl)benzyl)-3-(4-methylpiperazin-1-yl)propan-1-amine (2{6,9}; X=H, m=3, n=0, Z= 4-methylpiperazin-1-yl, Y=piperidin-1-yl:

As for 3{*11*,*9*}, but using 0.96 g (3.5 mmol) of 9{6} and 0.36 g (3.5 mmol) of 1-aminopiperidine {*9*}. 1.21 g (3.4 mmol, 97%) of a yellow oil 2{*6,9*} are obtained. IR (film): ν (cm⁻¹) 3286, 2935, 2875, 2853, 2794, 1591, 1451, 1357, 1015. ¹H-NMR (300 MHz, CDCl₃): δ (ppm) 7.55 (d, 2H, *J*=8.1 Hz, Ph), 7.54 (s, 1H, CH=N), 7.27 (d, 2H, *J*=8.1 Hz, Ph), 3.77 (s, 2H, CH₂-Ph), 3.15 (t, 4H, *J*=5.7 Hz, CH₂-N), 2.67 (t, 2H, *J*=6.9 Hz, CH₂-N), 2.42 (bs, 8H, CH₂-N), 2.40 (t, 2H, *J*=6.9 Hz, CH₂-N), 2.27 (s, 3H, CH₃), 2.16 (bs, 1H, NH), 1.79-1.66 (m, 6H, CH₂), 1.54 (m, 2H, CH₂). ¹³C-NMR (75 MHz, CDCl₃): δ (ppm) 139.7, 135.4, 134.5, 128.1, 125.9, 57.0, 55.1, 53.7, 53.2, 52.1, 48.1, 46.0, 26.9, 25.3, 24.2. MS (IE): m/z 358.3 [M+1H]⁺, 273.2, 201.1, 113.0, 84.0. HRMS: Calculated for C₂₁H₃₅N₅: 357.2892. Obtained: 357.2903.

### Obtaining N-(4-((piperidin-1-ylimino)methyl)benzyl)-3-morpholinopropan-1-amine (2{8,9}; X=H, m=3, n=0, Z= morpholino, Y= piperidin-1-yl):

As for 3{*11,9*}, but using 0.93 g (3.6 mmol) of 9{*8*} and 0.37 g (3.6 mmol) of 1-aminopiperidine {*9*}. 1.21 g (3.5 mmol, 99%) of a yellow oil 2{*8,9*} are obtained. IR (CHCl₃ evaporated film): ν (cm⁻¹) 3298, 2936, 2853, 2807, 1591, 1451, 1118, 861, 762. ¹H-NMR (300 MHz, CDCl₃): δ (ppm) 7.55 (d, 2H, *J*=8.1 Hz, Ph), 7.54 (s, 1H, CH=N), 7.27 (d, 2H, *J*=8.1 Hz, Ph), 3.77 (s, 2H, CH₂-Ph), 3.69 (t, 4H, *J*=4.7 Hz, CH₂-O), 3.15 (t, 4H, *J*=5.6 Hz, CH₂-N), 2.67 (t, 2H, *J*=6.9 Hz, CH₂-N), 2.44-2.37 (m, 6H, CH₂-N), 1.99 (bs, 1H, NH), 1.79-1.65 (m, 6H, CH₂), 1.54 (m, 2H, *J*=5.7 Hz, CH₂). ¹³C-NMR (75 MHz, CDCl₃): δ (ppm) 139.8, 135.4, 134.4, 128.1, 125.9, 67.0, 57.4, 53.8, 52.1, 47.9, 26.6, 25.3, 24.2. MS (IE): m/z 345.2 [M+1H]⁺, 344.2, 260.1, 100.0. HRMS: Calculated for C₂₀H₃₂N₄O: 344.2576. Obtained: 344.2580.

### Obtaining N-(4-((2,6-dimethylpiperidin-1-ylimino)methyl)benzyl)-3-(pyrrolidin-1-yl)propan-1-amine (2{2,10}; X=H, m=3, n=0, Z=pyrrolidin-1-yl, Y=2,6-dimethylpiperidin-1-yl):

As for 3{*11*,*9*}, but using 1.00 g (4.1 mmol) of 9{*2*} and 0.58 g (4.1 mmol) of 1-amino-2,6-dimethylpiperidine {*10*}. 1.18 g (3.3 mmol, 81%) of a yellow oil 2{*2*,*10*} are obtained. IR (film): ν (cm⁻¹) 3283, 2961, 2931, 2872, 2794, 1625, 1449, 1369, 1048. ¹H-NMR (300 MHz, CDCl₃): δ (ppm) 8.06 (s, 1H, CH=N), 7.64 (d, 2H, *J*=8.1 Hz, Ph), 7.33 (d, 2H, *J*=8.1 Hz, Ph), 3.81 (s, 2H, CH₂-Ph), 3.07 (bs, 2H, CH), 2.69 (t, 2H, *J*=6.9 Hz, CH₂-N), 2.51 (m, 6H, CH₂-N), 2.08 (bs, 1H, NH), 1.79-1.69 (m, 10H, CH₂), 1.53 (m, 2H, CH₂), 1.00 (d, 6H, *J*=6.3 Hz, CH₃). ¹³C-NMR (75 MHz, CDCl₃): δ (ppm) 152.4, 141.7, 133.7, 128.1, 127.3, 57.3, 54.8, 54.2, 53.7, 48.0, 32.9, 29.1, 23.5, 21.5, 20.6. MS (IE): m/z 356.3 [M]⁺, 244.2, 98.2, 84.1. HRMS: Calculated for C₂₂H₃₆N₄: 356.2940. Obtained: 356.2934.

### Obtaining N-(4-((4-methylpiperazin-1-ylimino)methyl)benzyl)-3-(pyrrolidin-1-yl)propan-1-amine (2{2,6}; X=H, m=3, n=0, Z=pyrrolidin-1-yl, Y=4-methylpiperazin-1-yl):

As for 3{*11*,*9*}, but using 1.03 g (4.2 mmol) of 9{*2*} and 0.50 g (4.2 mmol) of 3-(4-methylpiperazin-1-yl)propan-1-amine {*6*}. 1.43 g (4.2 mmol, 100%) of a yellow oil 2{*2,6*} are obtained. IR (film): ν (cm⁻¹) 3288, 2937, 2876, 2794, 1592, 1452, 1365, 1355. ¹H-NMR (300 MHz, CDCl₃): δ (ppm) 7.55 (d, 2H, *J*=8.1 Hz, Ph), 7.54 (s, 1H, CH=N), 7.28 (d, 2H, *J*=8.1 Hz, Ph), 3.78 (s, 2H, CH₂-Ph), 3.21 (t, 4H, *J*=5.1 Hz, CH₂-N), 2.70 (t, 2H, *J*=6.6 Hz, CH₂-N), 2.62 (t, 4H, *J*=5.1Hz, CH₂-N), 2.49 (m, 6H, CH₂-N), 2.35 (s, 3H, CH₃), 2.02 (bs, 1 H, NH), 1.76 (m, 6H, CH₂). ¹³C-NMR (75 MHz, CDCl₃): δ (ppm) 140.3, 135.8, 134.8, 128.1, 126.0, 54.8, 54.5, 54.3, 53.7, 51.0, 48.0, 46.0, 29.2, 23.5. MS (IE): m/z 343.2 [M]⁺, 244.2, 216.2, 99.2, 84.2. HRMS: Calculated for C₂₀H₃₃N₅: 343.2736. Obtained: 343.2736.

### Obtaining N-(4-((4-methylpiperazin-1-ylimino)methyl)benzyl)-3-(1H-imidazol-1-yl)propan-1-amine (2{3,6}; X=H, m=3, n=0, Z=1H-imidazol-1-yl, Y=4-methylpiperazin-1-yl):

As for 3{*11*,*9*}, but using 0.92 g (3.8 mmol) of 9{*3*} and 0.45 g (3.8 mmol) of 3-(4-methylpiperazin-1-yl)propan-1-amine {*6*}. 1.23 g (3.6 mmol, 96%) of a yellow oil 2{*3,6*} are obtained. IR (film): ν (cm⁻¹) 3282, 3104, 2937, 2880, 2828, 2797, 1592, 1452, 1365, 1356, 999. ¹H-NMR (300 MHz, CDCl₃): δ (ppm) 7.56 (d, 2H, *J*=8.1 Hz, CH₂-Ph), 7.55 (s, 1 H, CH=N), 7.45 (s, 1 H, CH), 7.27 (d, 2H, *J*=8.1 Hz, CH₂-Ph), 7.04 (s, 1H, CH), 6.89 (s, 1H, CH), 4.04 (t, 2H, *J*=6.9 Hz, CH₂-N), 3.75 (s, 2H, CH₂-Ph), 3.22 (t, 4H, *J*=5.1 Hz, CH₂-N), 2.64-2.58 (m, 6H, CH₂-N), 2.36 (s, 3H, CH₃), 1.92 (m, 2H, *J*=6.9 Hz, CH₂), 1.70 (bs, 1H, NH). ¹³C-NMR (75 MHz, CDCl₃): δ (ppm) 140.0, 137.0, 135.6, 135.1, 129.2, 128.1, 126.1, 118.7, 54.5, 53.7, 51.0, 45.9, 45.6, 44.7, 31.4. MS (IE): m/z 341.3 [M+1 H]⁺, 98.2, 56.0. HRMS: Calculated for C₁₉H₂₈N₆: 340.2375. Obtained: 340.2374.

### Obtaining N-(4-((4-methylpiperazin-1-ylimino)methyl)benzyl)-3-(2-methylpiperidin-1-yl)propan-1-amine (2{5,6}; X=H, m=3, n=0, Z=2-methylpiperidin-1-yl, Y=4-methylpiperazin-1-yl):

As for 3{*11*,*9*} but using 0.93 g (3.4 mmol) of 9{*5*} and 0.40 g (3.4 mmol) of 3-(4-methylpiperazin-1-yl)propan-1-amine {*6*}. 1.05 g (2.8 mmol, 84%) of a red oil 2{5,6} are obtained. IR (film): ν (cm⁻¹) 3279, 2932, 2881, 2841, 2795, 1593, 1452, 1366, 1000. ¹H-NMR (300 MHz, CDCl₃): δ (ppm) 7.56 (d, 2H, *J*=8.1 Hz, CH₂-Ph), 7.55 (s, 1H, CH=N), 7.29 (d, 2H, *J*=8.1 Hz, CH₂-Ph), 3.77 (s, 2H, CH₂-Ph), 3.21 (t, 4H, *J*=5.1 Hz, CH₂-N), 2.87 (m, 1H, CH), 2.73 (m, 1H, CH₂), 2.65-2.60 (m, 7H, CH₂-N), 2.36 (s, 3H, CH₃), 2.29 (m, 1 H, CH₂), 2.11 (m, 1 H, CH₂), 1.95 (bs, 1 H, NH), 1.73-1.48 (m, 6H, CH₂), 1.27 (m, 2H, CH₂), 1.04 (d, 3H, *J*=6.0 Hz, CH₃). ¹³C-NMR (75 MHz, CDCl₃): δ (ppm) 140.3, 135.8, 134.8, 128.2, 126.1, 56.0, 54.5, 54.0, 52.2, 52.1, 51.1, 48.3, 46.0, 34.7, 26.2, 25.8, 24.0, 19.1. MS (IE): m/z 372.4 [M+1 H]⁺, 371.4, 272.3, 216.2, 112.2, 99.2. HRMS: Calculated for C₂₂H₃₇N₅: 371.3049. Obtained: 371.3053.

### Obtaining N-(4-((4-methylpiperazin-1-ylimino)methyl)benzyl)-3-(4-methylpiperazin-1-yl)propan-1-amine (2{6,6}; X=H, m=3, n=0, Z=4-methylpiperazin-1-yl, Y=4-methylpiperazin-1-yl):

As for 3{*11*,*9*}, but using 0.93 g (3.4 mmol) of 9{*6*} and 0.40 g (3.4 mmol) of 3-(4-methylpiperazin-1-yl)propan-1-amine {6}. 1.25 g (3.4 mmol, 99%) of a yellowish oil 2{6,6} are obtained. IR (film): ν (cm⁻¹) 3283, 2936, 2877, 2836, 2794, 2768, 1593, 1453, 1365, 1356, 999. ¹H-NMR (300 MHz, CDCl₃): δ (ppm) 7.55 (d, 2H, *J*=8.1 Hz, CH₂-Ph), 7.55 (s, 1H, CH=N), 7.28 (d, 2H, *J*=8.1 Hz, CH₂-Ph), 3.77 (s, 2H, CH₂-Ph), 3.21 (t, 4H, *J*=5.1 Hz, CH₂-N), 2.66 (t, 2H, *J*=6.9 Hz, CH₂-N), 2.62 (t, 4H, *J*=5.1 Hz, CH₂-N), 2.42 (bs, 10H, CH₂-N), 2.40 (t, 2H, *J*=6.9 Hz, CH₂-N), 2.36 (s, 3H, CH₃), 2.71 (s, 3H, CH₃), 1.82 (bs, 1 H, NH), 1.70 (m, 2H, *J*=6.9 Hz, CH₂). ¹³C-NMR (75 MHz, CDCl₃): δ (ppm) 140.2, 135.8, 134.8, 128.1, 126.0, 57.0, 55.1, 54.5, 53.7, 53.2, 51.0, 48.0, 46.0, 45.9, 26.9. MS (IE): m/z 373.4 [M+1H]⁺, 372.3, 273.3, 216.2, 113.2, 99.2, 56.0. HRMS: Calculated for C₂₁H₃₆N₆: 372.3001. Obtained: 372.2990.

### Obtaining N-(4-((4-methylpiperazin-1-ylimino)methyl)benzyl)-3-morpholinopropan-1-amine (2{8,6}; X=H, m=3, n=0, Z=morpholino, Y=4-methylpiperazin-1-yl):

As for 3{*11*,*9*}, but using 0.89 g (3.4 mmol) of 9{*8*} and 0.40 g (3.4 mmol) of 3-(4-methylpiperazin-1-yl)propan-1-amine {6}. 1.22 g (3.4 mmol, 100%) of a yellow oil 2{*6*,*6*} are obtained. IR (CHCl₃ evaporated film): ν (cm⁻¹) 3293, 2939, 2888, 2844, 2799, 2592, 1453, 1364, 1356, 1118, 806. ¹H-NMR (300 MHz, CDCl₃): δ (ppm) 7.56 (s, 1H, CH=N), 7.56 (d, 2H, *J*=8.1 Hz, Ph), 7.28 (d, 2H, *J*=8.1 Hz, Ph), 3.77 (s, 2H, CH₂-Ph), 3.69 (t, 4H, *J*=4.7 Hz, CH₂-O), 3.21 (t, 4H, *J*=5.1 Hz, CH₂-N), 2.67 (t, 2H, *J*=6.9 Hz, CH₂-N), 2.62 (t, 4H, *J*=5.1 Hz, CH₂-N), 2.44-2.37 (m, 6H, CH₂-N), 2.35 (s, 3H, CH₃), 2.05 (bs, 1 H, NH), 1.70 (m, 2H, *J*=6.9 Hz, CH₂). ¹³C-NMR (75 MHz, CDCl₃): δ (ppm) 140.2, 135.7, 134.9, 128.1, 126.1, 66.9, 57.4, 54.5, 53.8, 51.0, 47.9, 45.9, 26.6. MS (IE): m/z 359.2 [M]⁺, 260.2, 231.2, 100.0. HRMS: Calculated for C₂₀H₃₃N₅O: 359.2685. Obtained: 359.2685.

## Claims

1. A compound of formula 1: 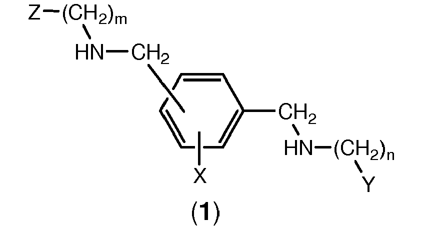 wherein:
the substituent -CH₂-NH-(CH₂)ₘ-Z is *meta* or *para* to the substituent -CH₂-NH-(CH₂)ₙ-Y wherein m and n, which may be identical or different, may have the values 0, 2, 3, 4, 5 and 6;
Z and Y, which may be identical or different, represent a nitrogenated heterocyclic system bonded by nitrogen (the corresponding m or n being greater than or equal to 2) or by one of the ring carbons; a substituted nitrogenated heterocyclic system bonded by nitrogen (the corresponding m or n being greater than or equal to 2) or by one of the ring carbons; an NR¹R² group (the corresponding m or n being greater than or equal to 2) wherein R¹ and R² are independently selected from among hydrogen, C₁-C₁₂ alkyl, substituted alkyl, C₃-C₁₂ aryl, substituted C₃-C₁₂ aryl, cycloalkyl and substituted cycloalkyl
X is selected from the group consisting of hydrogen, C₁-C₁₂ alkyl, substituted alkyl, C₃-C₁₂ aryl, amino, alkylamino, nitro, hydroxy, alkoxy, halogen, carboxy or carboxamido;
and pharmaceutically acceptable salts, hydrates, solvates, esters and metal complexes of said structure.

2. A compound of formula 2: 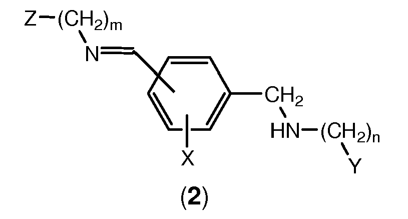 wherein:
the substituent -CH=NH-(CH₂)ₘ-Z is *meta* or *para* to the substituent -CH₂-NH-(CH₂)ₙ-Y wherein m and n, which may be identical or different, may have the values 0, 2, 3, 4, 5 and 6;
Z and Y, which may be identical or different, represent a nitrogenated heterocyclic system bonded by nitrogen (n being greater than or equal to 2) or by one of the ring carbons; a substituted nitrogenated heterocyclic system bonded by nitrogen (n being greater than or equal to 2) or by one of the ring carbons; an NR¹R² group (n being greater than or equal to 2) wherein R¹ and R² are independently selected from hydrogen, C1-C12 alkyl, substituted alkyl, C₃-C₁₂ aryl, substituted C3-C12 aryl, cycloalkyl and substituted cycloalkyl
X is selected from the group consisting of hydrogen, C1-C12 alkyl, substituted alkyl, C₃-C₁₂ aryl, amino, alkylamino, nitro, hydroxy, alkoxy, halogen, carboxy or carboxamido;
and pharmaceutically acceptable salts, hydrates, solvates, esters and metal complexes of said structure.

3. A compound of formula 3: 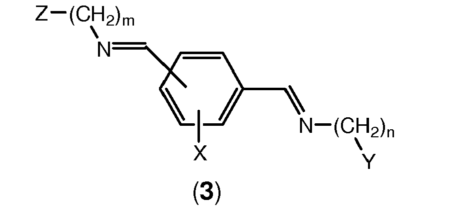 wherein:
the substituent -CH=NH-(CH₂)ₘ-Z is *meta* or *para* to the substituent -CH=NH-(CH₂)ₙ-Y wherein m and n, which may be identical or different, may have the values 0, 2, 3, 4, 5 and 6;
Z and Y, which may be identical or different, represent a nitrogenated heterocyclic system bonded by nitrogen or by one of the ring carbons; a substituted nitrogenated heterocyclic system bonded by nitrogen or by one of the ring carbons; an NR¹R² group wherein R¹ and R² are independently selected from hydrogen, C1-C12 alkyl, substituted alkyl, C₃-C₁₂ aryl, substituted C3-C12 aryl, cycloalkyl and substituted cycloalkyl
X is selected from the group consisting of hydrogen, C1-C12 alkyl, substituted alkyl, C₃-C₁₂ aryl, amino, alkylamino, nitro, hydroxy, alkoxy, halogen, carboxy or carboxamido;
and pharmaceutically acceptable salts, hydrates, solvates, esters and metal complexes of said structure.

4. A compound 1 according to claim 1, selected from the group consisting of:
*N*-(4-((2-(pyrrolidin-1-yl)ethylamino)methyl)benzyl)-2-(pyrrolidin-1-yl)ethylamine
*N*-(4-((3-(pyrrolidin-1-yl)propylamino)methyl)benzyl)-3-(pyrrolidin-1-yl)propan-1-amine
N-(4-((3-(1*H*-imidazol-1-yl)propylamino)methyl)benzyl)-3-(1*H*-imidazol-1-yl)propan-1-amine
*N*-(4-((3-(2-methylpiperidin-1-yl)propylamino)methyl)benzyl)-3-(2-methylpiperidin-1-yl)propan-1-amine
*N*-(4-((3-(4-methylpiperazin-1-yl)propylamino)methyl)benzyl)-3-(4-methylpiperazin-1-yl)propan-1-amine
*N*-(4-((3-morpholinopropylamino)methyl)benzyl)-3-morpholinopropan-1-amine
*N*-(4-((2-(pyrrolidin-1-yl)ethylamino)methyl)benzyl)-3-(pyrrolidin-1-yl)propan-1-amine
*N*-(4-((2-(pyrrolidin-1-yl)ethylamino)methyl)benzyl)-3-(1*H*-imidazol-1-yl)propan-1-amine
*N*-(4-((2-(pyrrolidin-1-yl)ethylamino)methyl)benzyl)-3-(4-methylpiperazin-1-yl)propan-1-amine
*N*-(4-((2-(pyrrolidin-1-yl)ethylamino)methyl)benzyl)-3-morpholinopropan-1-amine
*N*-(4-((3-(1*H*-imidazol-1-yl)propylamino)methyl)benzyl)-3-(pyrrolidin-1-yl)propan-1-amine
*N*-(4-((2-(piperidin-1-yl)ethylamino)methyl)benzyl)-3-(pyrrolidin-1-yl)propan-1-amine
*N*-(4-((3-(pyrrolidin-1-yl)propylamino)methyl)benzyl)-3-(2-methylpiperidin-1-yl)propan-1-amine
*N*-(4-((3-(4-methylpiperazin-1-yl)propylamino)methyl)benzyl)-3-(pyrrolidin-1-yl)propan-1-amine
*N*-(4-((2-morpholinoethylamino)methyl)benzyl)-3-(pyrrolidin-1-yl)propan-1-amine
*N*-(4-((3-(pyrrolidin-1-yl)propylamino)methyl)benzyl)-3-morpholinopropan-1-amine
*N*-(4-((2-(piperidin-1-yl)ethylamino)methyl)benzyl)-3-(1*H*-imidazol-1-yl)propan-1-amine
N-(4-((3-(1*H*-imidazol-1-yl)propylamino)methyl)benzyl)-3-(2-methylpiperidin-1-yl)propan-1-amine
*N*-(4-((3-(4-methylpiperazin-1-yl)propylamino)methyl)benzyl)-3-(1*H*-imidazol-1-yl)propan-1-amine
*N*-(4-((2-morpholinoethylamino)methyl)benzyl)-3-(1*H*-imidazol-1-yl)propan-1-amine
*N*-(4-((3-morpholinopropylamino)methyl)benzyl)-3-(1*H*-imidazol-1-yl)propan-1-amine
*N*-(4-((2-(piperidin-1-yl)ethylamino)methyl)benzyl)-3-(4-methylpiperazin-1-yl)propan-1-amine
*N*-(4-((2-(piperidin-1-yl)ethylamino)methyl)benzyl)-3-morpholinopropan-1-amine
N-(4-((3-(4-methylpiperazin-1 -yl)propylamino)methyl)benzyl)-3-(2-methylpiperidin-1-yl)propan-1-amine
*N*-(4-((2-morpholinoethylamino)methyl)benzyl)-3-(2-methylpiperidin-1-yl)propan-1-amine
*N*-(4-((3-morpholinopropylamino)methyl)benzyl)-3-(2-methylpiperidin-1-yl)propan-1-amine
*N*-(4-((2-morpholinoethylamino)methyl)benzyl)-3-(4-methylpiperazin-1-yl)propan-1-amine
*N*-(4-((3-morpholinopropylamino)methyl)benzyl)-3-(4-methylpiperazin-1-yl)propan-1-amine
*N*-(4-((2-morpholinoethylamino)methyl)benzyl)-3-morpholinopropan-1-amine

5. A compound 2 according to claim 2, selected from the group consisting of:
*N*-(4-((2,6-dimethylpiperidin-1-ylimino)methyl)benzyl)-2-(pyrrolidin-1-yl)ethylamine
*N*-(4-((2,6-dimethylpiperidin-1-ylimino)methyl)benzyl)-3-(1*H*-imidazol-1-yl)propan-1-amine
*N*-(4-((2,6-dimethylpiperidin-1-ylimino)methyl)benzyl)-3-(4-methylpiperazin-1-yl)propan-1-amine
*N*-(4-((2,6-dimethylpiperidin-1-ylimino)methyl)benzyl)-3-morpholinopropan-1-amine
*N*-(4-((4-methylpiperazin-1-ylimino)methyl)benzyl)-2-(pyrrolidin-1-yl)ethanamine
*N*-(4-((piperidin-1-ylimino)methyl)benzyl)-3-(pyrrolidin-1-yl)propan-1-amine
*N*-(4-((piperidin-1-ylimino)methyl)benzyl)-3-(1*H*-imidazol-1-yl)propan-1-amine
*N*-(4-((piperidin-1-ylimino)methyl)benzyl)-3-(2-methylpiperidin-1-yl)propan-1-amine
*N*-(4-((piperidin-1-ylimino)methyl)benzyl)-3-(4-methylpiperazin-1-yl)propan-1-amine
*N*-(4-((piperidin-1-ylimino)methyl)benzyl)-3-morpholinopropan-1-amine
*N*-(4-((2,6-dimethylpiperidin-1-ylimino)methyl)benzyl)-3-(pyrrolidin-1-yl)propan-1-amine
*N*-(4-((4-methylpiperazin-1-ylimino)methyl)benzyl)-3-(pyrrolidin-1-yl)propan-1-amine
*N*-(4-((4-methylpiperazin-1-ylimino)methyl)benzyl)-3-(1*H*-imidazol-1-yl)propan-1-amine
*N*-(4-((4-methylpiperazin-1-ylimino)methyl)benzyl)-3-(2-methylpiperidin-1-yl)propan-1-amine
*N*-(4-((4-methylpiperazin-1-ylimino)methyl)benzyl)-3-(4-methylpiperazin-1-yl)propan-1-amine
*N*-(4-((4-methylpiperazin-1-ylimino)methyl)benzyl)-3-morpholinopropan-1-amine

6. A compound 3 according to claim 3, selected from the group consisting of:
((4-(*N*-(piperidin-1-yl)imino)methyl)phenyl)-N-(piperidin-1-yl)methanimine
((4-(*N*-(2,6-dimethylpiperidin-1-yl)imino)methyl)phenyl)-N-(2,6-dimethylpiperidin-1-yl)methanimine
((4-(*N*-(4-methylpiperazin-1-yl)imino)methyl)phenyl)-N-(piperidin-1-yl)methanimine

7. A process for preparing a compound of formula (1) as has been defined in claim 1, comprising:
a) treating a dialkoxymethylbenzaldehyde of general structure (4) with an amino derivative of general structure NH₂-(CH₂)ₙ-Y in the presence of a reducing agent to yield a compound of formula (6), optionally, isolating the intermediate imine (5) in the presence of a dehydrating agent. 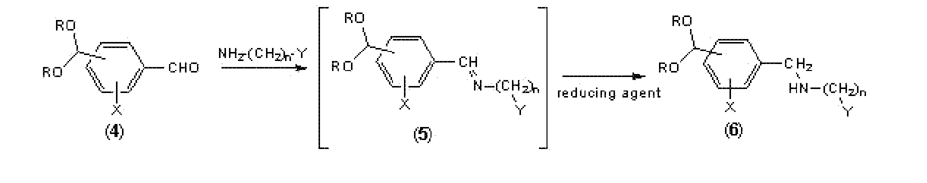
b) deprotecting the acetal present in a compound of structure (6) to obtain the corresponding aldehyde of structure (7) 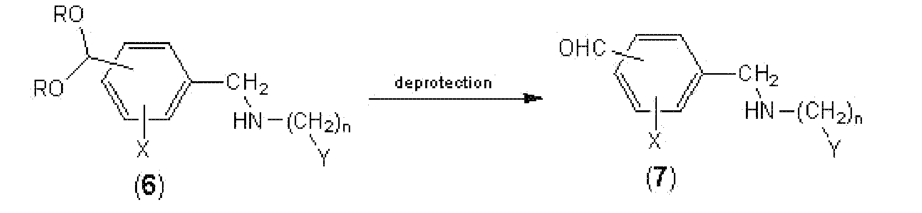
c) treating an aldehyde of structure (7) with an amino derivative of general structure NH₂-(CH₂)ₘ-Z in the presence of a reducing agent to obtain the corresponding compound of structure (1), optionally, isolating the intermediate imine (2) in the presence of a dehydrating agent.

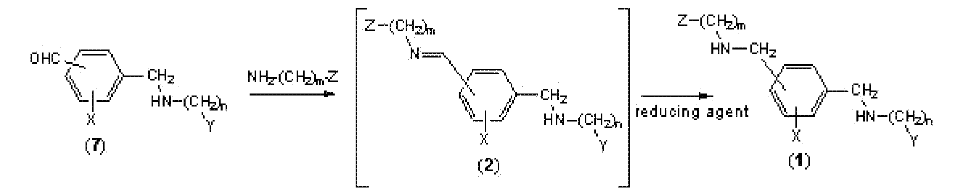

8. A process for preparing a compound of formula (1) as has been defined in claim 1, comprising treating an imine of formula (2) with a reducing agent.

9. A process for preparing a compound of formula (1) as has been defined in claim 1, comprising:
a) treating a dialkoxymethylbenzaldehyde of general structure (4) with an amino derivative of general structure NH₂-(CH₂)ₙ-Y in the presence of a dehydrating agent to yield a compound of formula (5). 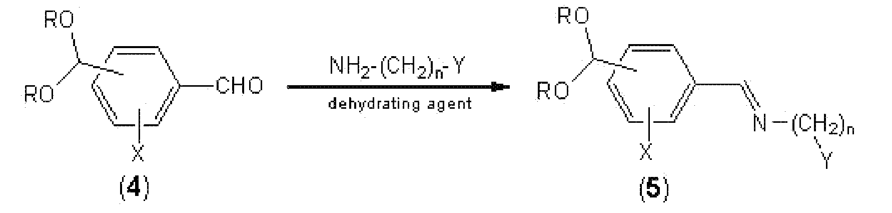
b) deprotecting the acetal present in a compound of structure (5) to obtain the corresponding aldehyde of structure (8). 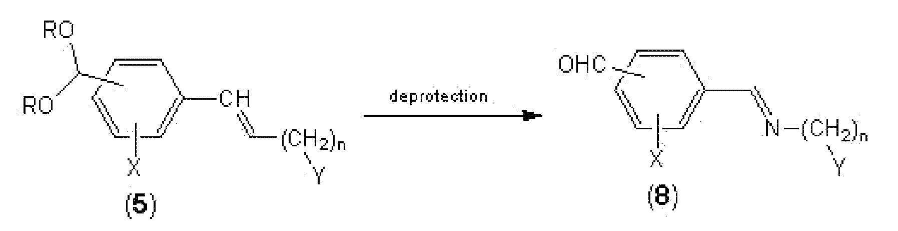
c) treating an aldehyde of structure (8) with an amino derivative of general structure NH₂-(CH₂)ₘ-Z in the presence of a dehydrating agent to obtain the corresponding compound of structure (3). 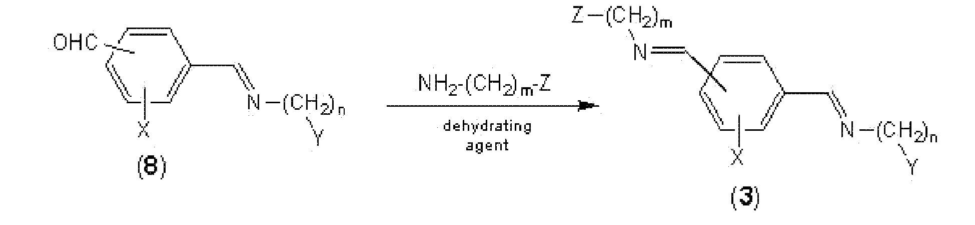
d) treating a compound of structure (3) with a reducing agent to obtain the corresponding compound of structure (1).

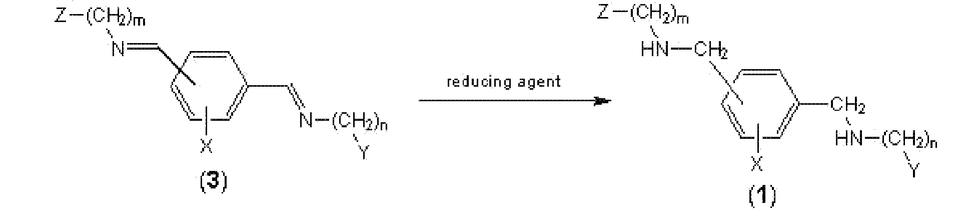

10. A process for preparing a compound of formula (1) as has been defined in claim 1, comprising treating a diimine of formula (3) with a reducing agent.

11. A process for preparing a compound of formula (1 a), (1) wherein X=H and the substituents are in *para* according to claim 7, wherein the dialkoxymethylbenzaldehyde is 4-(diethoxymethyl)benzaldehyde (4a).

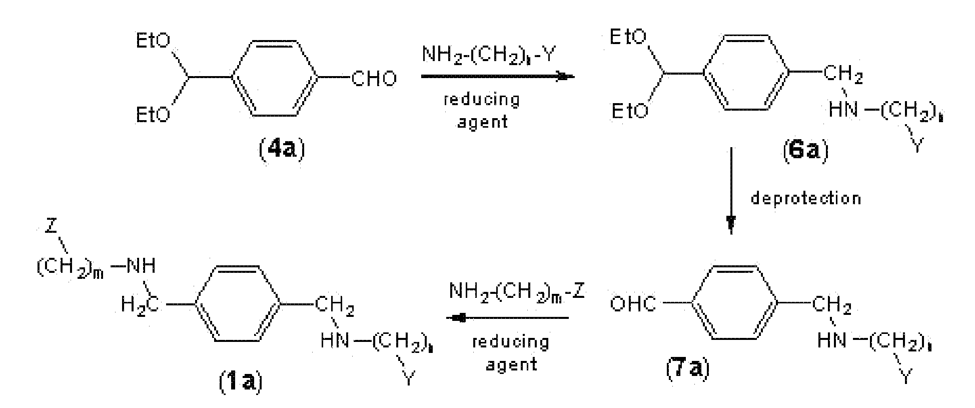

12. A process for preparing a compound of formula (1 a), (1) wherein X=H and the substituents are in *para* according to claim 9 wherein the dialkoxymethylbenzaldehyde is 4-(diethoxymethyl)benzaldehyde (4a).

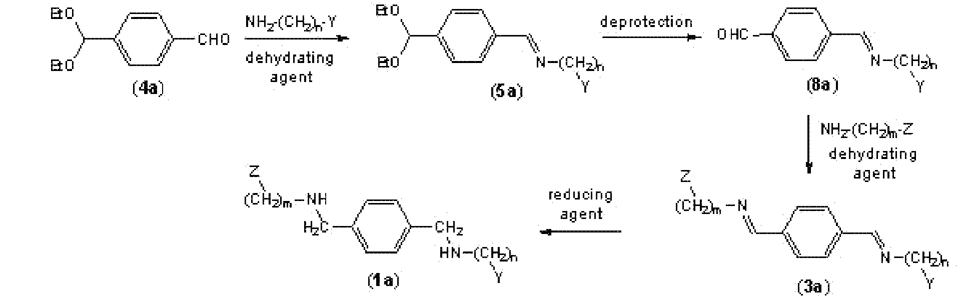

13. A process for preparing a compound of formula (1b), (1) as has been defined in claim 1 wherein m = n and Z = Y, comprising treating a dialdehyde of general structure (9) with an amino derivative of general structure NH₂-(CH₂)ₘ-Z in the presence of a reducing agent to yield a compound of formula (1b), optionally isolating the intermediate diimine (3b) in the presence of a dehydrating agent.

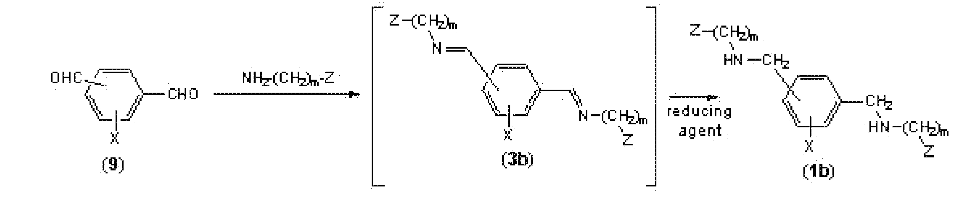

14. A process for preparing a compound of formula (1c), (1b) wherein X=H and the substituents are in *para* according to claim 13, wherein the dialdehyde is terephthaldehyde (9a).

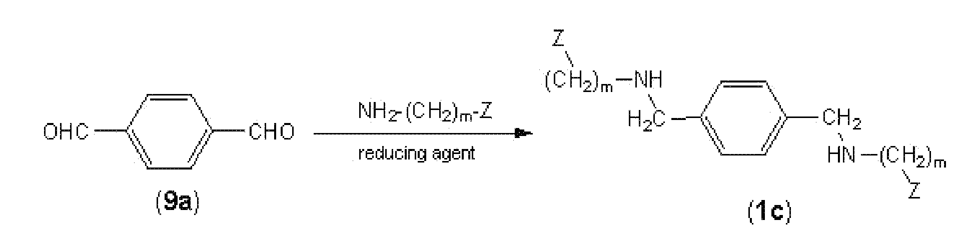

15. A process for preparing a compound of formula (2) as has been defined in claim 2, comprising:
a) treating a dialkoxymethylbenzaldehyde of general structure (4) with an amino derivative of general structure NH₂-(CH₂)ₙ-Y in the presence of a reducing agent to yield a compound of formula (6), optionally isolating the intermediate imine (5) in the presence of a dehydrating agent. 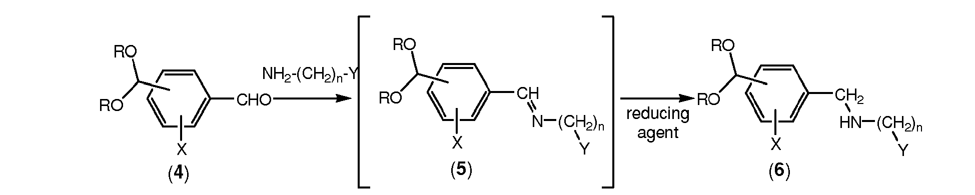
b) deprotecting the acetal present in a compound of structure (6) to obtain the corresponding aldehyde of structure (7). 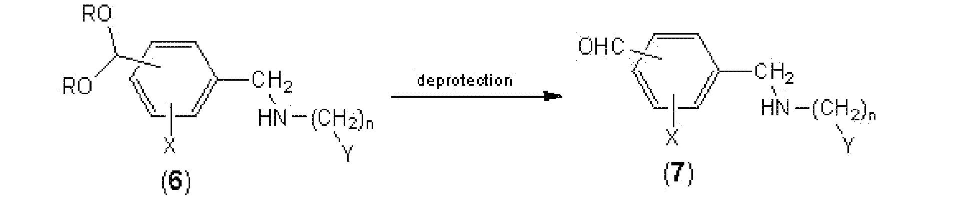
c) treating an aldehyde of structure (7) with an amino derivative of general structure NH₂-(CH₂)ₘ-Z in the presence of a dehydrating agent to obtain the corresponding compound of structure (2).

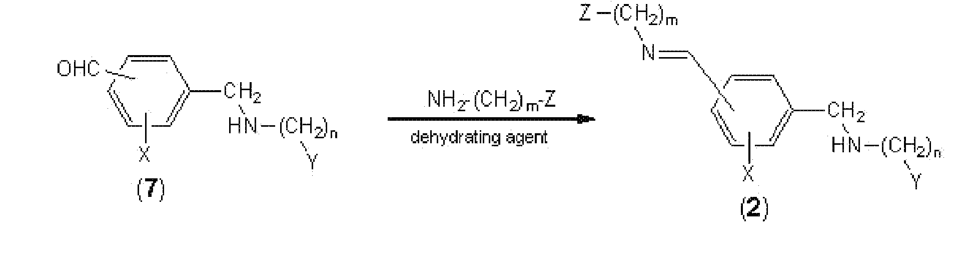

16. A process for preparing a compound of formula (2a), (2) wherein X=H and the substituents are in *para* according to claim 15, wherein the dialkoxymethylbenzaldehyde is 4-(diethoxymethyl)benzaldehyde (4a).

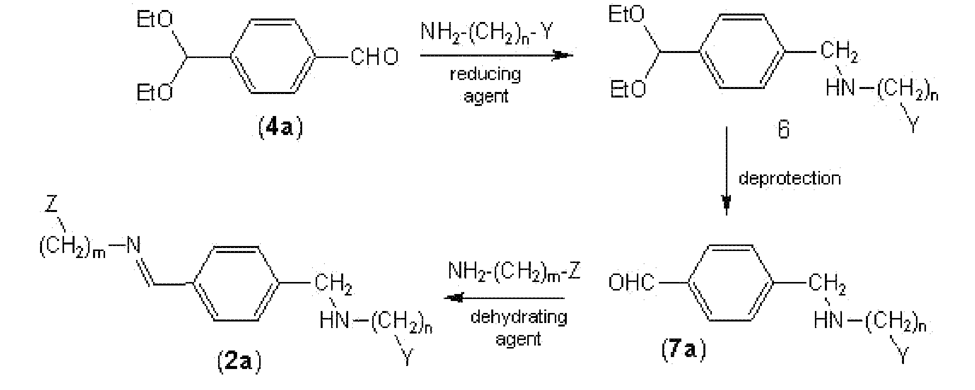

17. A process for obtaining a compound of formula (2d), compound (2) wherein n = 0, Y is bonded by nitrogen and m ≥ 2 if Z is bonded by nitrogen, comprising:
a) treating a dialdehyde of general structure (9) with a hydrazine of general structure NH₂-Y in the presence of a dehydrating agent to yield a compound of formula (10). 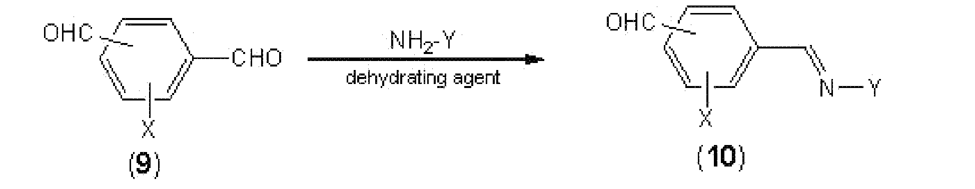
b) treating the compound (10) with an amino derivative of general structure NH₂-(CH₂)ₘ-Z (wherein m ≥ 2 if Z is bonded by nitrogen) in the presence of a reducing agent, optionally, isolating the intermediate diimine (3d) in the presence of a dehydrating agent.

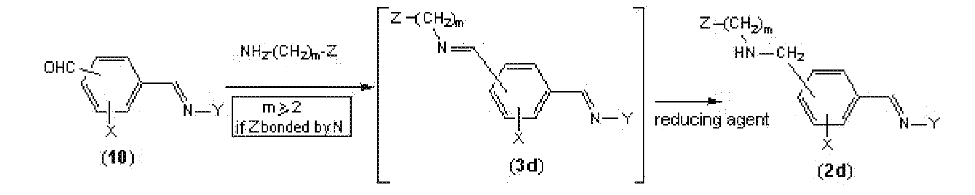

18. A process for preparing a compound of formula (3) as has been defined in claim 3, comprising:
a) treating a dialkoxymethylbenzaldehyde of general structure (4) with an amino derivative of general structure NH₂-(CH₂)ₙ-Y in the presence of a dehydrating agent to yield a compound of formula (5). 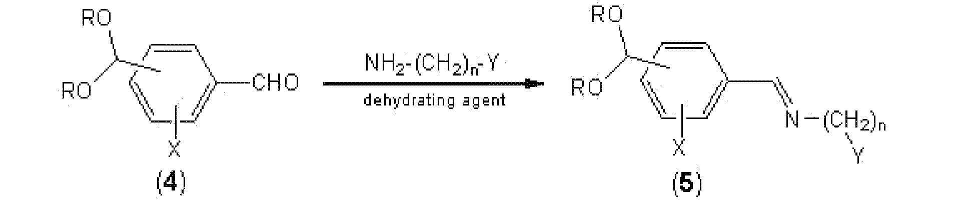
b) deprotecting the acetal present in a compound of structure (5) to obtain the corresponding aldehyde of structure (8). 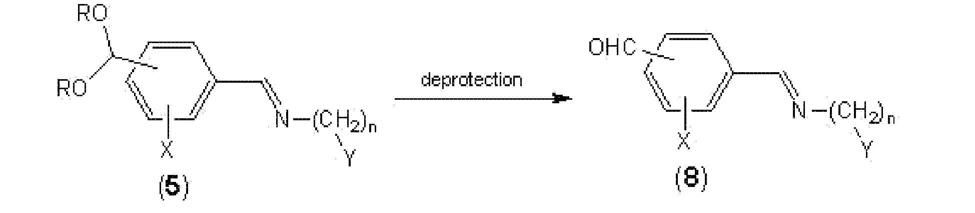
c) treating an aldehyde of structure (8) with an amino derivative of general structure NH₂-(CH₂)ₘ-Z in the presence of a dehydrating agent to obtain the corresponding compound of structure (3).

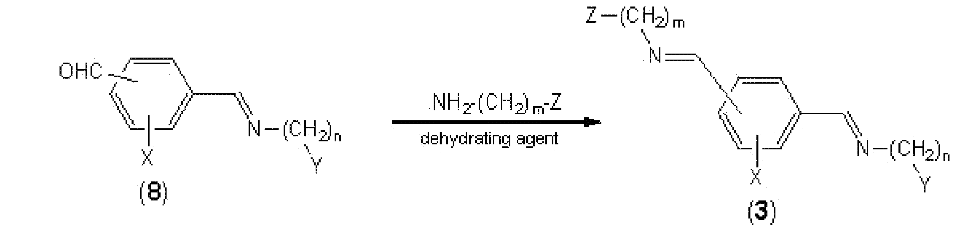

19. A process for preparing a compound of formula (3b), compound (3) wherein m = n and Z = Y, comprising treating a dialdehyde of general structure (9) with an amino derivative of general structure NH₂-(CH₂)ₘ-Z in the presence of a dehydrating agent to yield a compound of formula (3b).

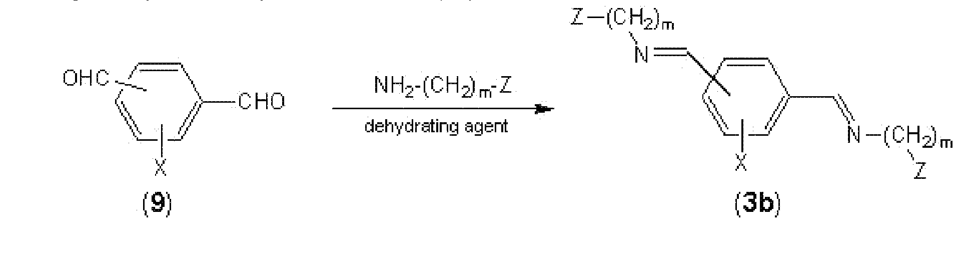

20. A process for preparing a compound of formula (3c), (3b) wherein X=H and the substituents are in *para* according to claim 19, wherein the dialdehyde is terephthaldehyde (9a).

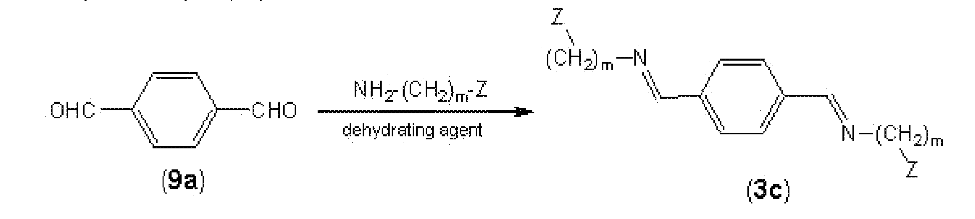

21. A process for preparing a compound of formula (3d), compound (3) wherein n= 0 and Y is bonded by nitrogen, comprising:
a) treating a dialdehyde of general structure (9) with a hydrazine of general structure NH₂-Y in the presence of a dehydrating agent to yield a compound of formula (10). 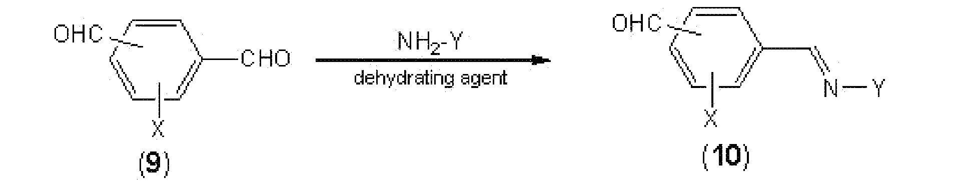
b) treating an aldehyde of structure (10) with an amino derivative of general structure NH₂-(CH₂)ₘ-Z in the presence of a dehydrating agent to obtain the corresponding compound of structure (3d)

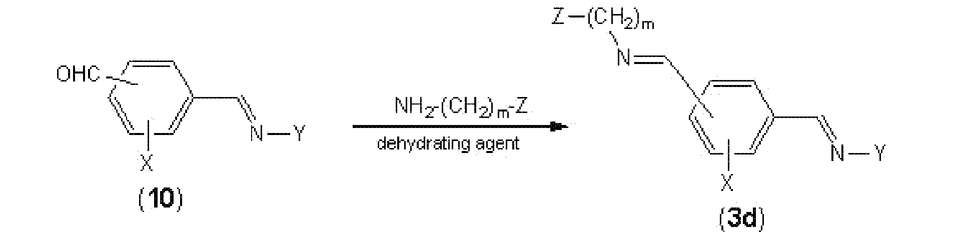

22. A process for preparing a compound of formula (3e), (3d) wherein X=H and the substituents are in *para* according to claim 21. wherein the dialdehyde is terephthaldehyde (9a).

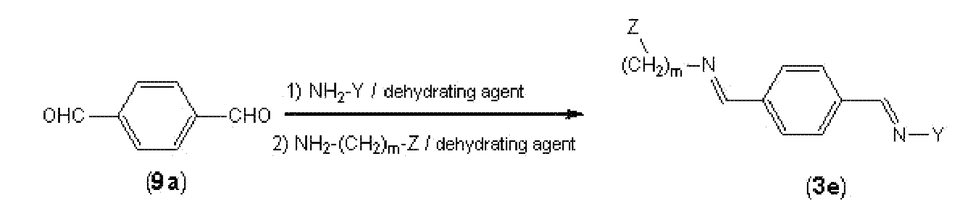

23. A process for preparing a compound of formula general (1) or (2) according to claims 7, 8, 9, 10, 11, 12, 13, 14, 15, 16 and 17, wherein the reducing agent is selected from among sodium borohydride and sodium cyanoborohydride.

24. A process for obtaining a compound of formula general (1), (2) or (3) according to claims 7, 9, 12, 13, 15, 16, 17, 18, 19, 20, 21 and 22, wherein the dehydrating agent are molecular sieves.

25. A compound of formula (7): 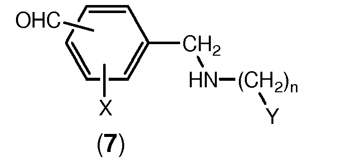 wherein:
the substituent -CH₂-NH-(CH₂)ₙ-Y is *meta* or *para* to the aldehyde group;
n may have the values 0, 2, 3, 4, 5 and 6;
Y represents a nitrogenated heterocyclic system bonded by nitrogen (n being greater than or equal to 2) or by one of the ring carbons; a substituted nitrogenated heterocyclic system bonded by nitrogen (n being greater than or
equal to 2) or by one of the ring carbons; an NR¹R² group (n being greater
than or equal to 2) wherein R¹ and R² are independently selected from among hydrogen, C₁-C₁₂ alkyl, substituted alkyl, C₃-C₁₂ aryl, substituted C₃-C₁₂ aryl, cycloalkyl and substituted cycloalkyl
X is selected from the group consisting of hydrogen, C₁-C₁₂ alkyl, substituted alkyl, C₃-C₁₂ aryl, amino, alkylamino, nitro, hydroxy, alkoxy, halogen, carboxy or carboxamido.

26. Use of the compounds of formulae (1), (2) and (3) to prepare a medicinal product for treating Acquired Immune Deficiency Syndrome (AIDS).
